# EUROPEAN PATENT APPLICATION

(11) **EP 3 979 161 A1**
(43) Date of publication of application: **06.04.2022**
(21) Application number: 20813804.0
(22) Date of filing: 12.05.2020
(51) Int. Cl.: G06Q 10/06

(54) **BUSINESS ASSISTANCE DEVICE, METHOD, AND PROGRAM**

(30) Priority: 31.05.2019 JP 2019102978
(71) Applicant: OMRON Corporation, Shiokoji-dori, Shimogyo-ku, Kyoto-shi Kyoto 600-8530 (JP)
(72) Inventor: KUBO, Fumihiko, Kyoto-shi, Kyoto 600-8234 (JP); EBINE, Hiroko, Kyoto-shi, Kyoto 600-8530 (JP); NOZAKI, Ritsuko, Kyoto-shi, Kyoto 600-8234 (JP)
(74) Representative: Horn Kleimann Waitzhofer Patentanwälte PartG mbB
(86) International application number: PCT/JP2020/018900
(87) International publication number: WO 2020/241229

(57) **Abstract**

This invention provides information for improving the job performance of an organization, thereby supporting the members of the organization. A job performance support apparatus includes an acquisition unit configured to acquire, for each department, index data including index contents corresponding to a plurality of first indices classified by at least two axes including an axis for classifying indices into a physical condition and a mental condition of a human resource belonging to an organization including at least one department, and an axis for classifying indices into a business management condition and an individual condition in the organization, and a second index concerning a job performance result of the department, a calculation unit configured to calculate, for each index, an evaluation value that evaluates the index contents from the index data, a first generation unit configured to designate a display mode of the evaluation value for each of the first indices and the second index, and generate, for each index, display data with which the evaluation value is associated, and a second generation unit configured to generate, for each department, support data including support measures corresponding to the first indices in accordance with a combination of the display data for the plurality of first indices and the second index.

## Description

### FIELD

Embodiments described herein relate generally to a job performance support apparatus using human resource exhibition measurement, a method, and a program.

### BACKGROUND

Currently, in business entities, workplace improvement activities that should be addressed company-wide, such as work style reform and health and productivity management, are accelerating, and there are increasing opportunities for requests to be generated from various aspects even in a workplace. Under these circumstances, there is increasing awareness that the role an organization should play includes not only requiring the members of the workplace to report the states of activities but also providing information and support that will lead to individual efforts for workplace reform to the members of the workplace for themselves.

As a part of workplace improvement, companies periodically carry out health examinations to manage the health of employees. In addition, stress checks are conducted to find an employee who may have a lot of stress in business at an early stage, and if it is considered necessary, an industrial physician or the like provides face-to-face guidance. As described above, at present, a company is widely required to do not only attendance management but also health examinations and stress checks for employees to improve labor environment.

To improve the labor environment for the employees, a measure is sometimes taken, in which, for example, a fatigue state is evaluated based on a health examination result and an attendance situation, and a manager refers to an evaluation table or the like, thereby improving the labor environment. Also, in addition to simply presenting a health examination result, a stress check result, and an attendance situation, the association between the results and situation may be grasped. The association shown by these is considered to be an important factor for improving the health condition or the working situation of an employee in the future.

Patent literature 1 discloses a labor environment improving system including a storage unit configured to store data representing, as a history, a result of each item of an attendance item group, a health examination item group, and a stress check item group, and an analysis unit configured to extract a second item as an associated item when one of the attendance item group, the health examination item group, and the stress check item group is a first item group, at least one item group other than the first item group is a second item group, data representing, as a history, a result of each first item of the first item group is first data, data representing, as a history, a result of each second item of the second item group is second data, and correlation of the second data to the first data satisfies a condition representing a level of the correlation.

### Citation List

### Patent Literature

Patent literature 1: JP-A 2017-107560 (KOKAI)

### SUMMARY

The above-described conventional apparatus extracts, as the associated item, the second item that satisfies the condition representing the level of the correlation. This makes it possible to easily grasp the first data of a specific first item and the second data of the associated item highly associated with the first item. In addition, it is possible to easily grasp data that are highly associated with each other in the attendance, health examinations, and stress checks.

However, since the conventional apparatus merely grasps the first data of the specific first item and the second data of the associated item highly associated with the first item, as described above, it is impossible to provide information and support that will lead to efforts for workplace reform to members. Also, according to this apparatus, it is impossible to grasp how the job performance of a company affects the environment of a workplace or individual health.

Hence, it is considered that providing a method of visualizing the performance of each department of an organization based on the business management situation of the organization and the health conditions of employees whose are the members of the organization, finding the relationship with the job performance of the organization, and guiding the organization to a sound and good condition as a whole will be more important in the future for an organization such as a company.

The present invention has been made in view of the above-described circumstances, and as one aspect, provides a job performance support apparatus configured to provide information for improving the job performance of an organization and support a member of the organization, a method, and a program.

In order to solve the above-described problem, the present disclosure employs the following configuration.

That is, a job performance support apparatus according to the first aspect of the present disclosure comprises an acquisition unit configured to acquire, for each department, index data including index contents corresponding to a plurality of first indices classified by at least two axes including an axis for classifying indices into a physical condition and a mental condition of a human resource belonging to an organization including at least one department, and an axis for classifying indices into a business management condition and an individual condition in the organization, and a second index concerning a job performance result of the department, a calculation unit configured to calculate, for each index, an evaluation value that evaluates the index contents from the index data, a first generation unit configured to designate a display mode of the evaluation value for each of the first indices and the second index, and generate, for each index, display data with which the evaluation value is associated, and a second generation unit configured to generate, for each department, support data including support measures corresponding to the first indices in accordance with a combination of the display data for the plurality of first indices and the second index.

In the above-described configuration, the acquisition unit acquires, for each of one or more departments of an organization, index data including index contents corresponding to a plurality of first indices and a second index concerning the job performance result of the department. Each first index is one of indices classified by at least two axes including an axis for classifying indices into a physical condition and a mental condition of a human resource belonging to the organization, and an axis for classifying indices into a business management condition and an individual condition in the organization. More specifically, the first indices include an index configured to show the physical condition of the human resource and the business management condition in the organization, an index configured to show the physical condition of the human resource and the individual condition in the organization, an index configured to show the mental condition of the human resource and the business management condition in the organization, and an index configured to show the mental condition of the human resource and the individual condition in the organization. Examples of the first indices are labor management (for example, attendance), health examination, engagement survey, and stress check. A plurality of indices may exist in correspondence with each of the first indices. In addition, the first indices may be classified not by only the two axes but by multiple axes including other axes. Note that the human resource indicates a human who can contribute to the result of the organization and exhibit high productivity.

The calculation unit calculates, from the index data acquired for each department, an evaluation value that evaluates the index contents of each index. The calculation unit compares a determination criterion set for each index with the index contents and calculates the evaluation value based on the comparison result. The evaluation value represents how much the index contents deserve evaluation (for example, whether the index contents are preferable contents), and is set to, for example, five levels. Note that the evaluation value is also called a score value.

The first generation unit designates the display mode representing a form to display the calculated evaluation values of the first indices and the second index, thereby deciding how to present the evaluation value of each index. The first generation unit then generates display data of all the calculated evaluation values of the indices based on the designated display modes. The display mode need only be a mode easy to understand at a glance. For example, a pie chart is divided and arranged for each first index, the second index is arranged at the center of the pie chart, and the evaluation value of each index is indicated by a color. The display mode is not limited to the pie chart, and may be another graph form. It is preferable that the first indices and the second index are discriminately displayed, and the association between the indices is easy to understand.

In accordance with the combination of the display data generated for each index by the first generation unit, the second generation unit associates each combination with support measures corresponding to the first indices for each department. The combination of the display data corresponds to the combination of the calculated evaluation values of the plurality of first indices and the second index. That is, in a department, support measures for the department are set based on the distribution of the evaluation values to the indices in the evaluation values of the plurality of first indices and the evaluation value of the second index. The second generation unit generates support data including the support measures for each department.

Hence, according to this disclosure, the current situation in a department of an organization can be analyzed by at least two axes including the axis for classifying indices into physical conditions and mental conditions of a human resource belonging to the organization and the axis for classifying indices into business management conditions and individual conditions. Also, according to the present disclosure, it is possible to clearly understand, for each department, how the plurality of indices are related to job performance. Since not only the understanding but also clearly showing a problem of a department and presenting a support measure for the problem is possible, it is possible to execute a detailed and appropriate measure for each personnel of a department. Hence, according to this aspect, it is possible to improve the job performance of the department and improve the job performance of the organization. As a result, the physical conditions and the mental conditions of the human resource can be guided to good conditions, and the business management conditions and the individual conditions can also be guided to good conditions. It can be expected that the job performance of the organization is improved, and the management conditions of the organization itself, the individual conditions of the members of the organization, and also the physical conditions and the mental conditions are guided to good conditions. When display data in which an evaluation value is associated with each index is time-serially listed, it is possible to easily and reliably analyze which index is improved or deteriorated in which manner. It is therefore possible to provide information for improving the job performance of the organization and support the members of the organization.

Furthermore, the job performance support apparatus according to the first aspect of the present disclosure need only be an apparatus capable of executing a program configured to acquire index data, calculate an evaluation value for each index, generate display data in which the display mode of the evaluation value is designated for each index, and generate support data including support measures corresponding to the first indices, and may be, for example, a wearable device (for example, a smartphone or a wristwatch-type wearable terminal) or a stationary device (for example, a personal computer). As for the department, for example, if the organization is a group company, a department may indicate a certain group company. Also, if the group company is defined as an organization, display data and support data may be generated for each index for a lot of departments included in the group company. That is, the terms "organization" and "department" are used in relative senses.

In the job performance support apparatus according to the second aspect of the present disclosure, the acquisition unit employs labor management and acquires labor management data as the first index reflecting the physical condition and the business management condition in the index data, employs a health examination and acquires health examination data as the first index reflecting the physical condition and the individual condition, employs an engagement survey and acquires engagement data as the first index reflecting the mental condition and the business management condition, and employs a stress check and acquires stress check data as the first index reflecting the mental condition and the individual condition.

In the above-described configuration, as the data acquired as the first indices, the acquisition unit defines examples of four indices that can be classified by four conditions. That is, the examples of four indices are labor management of the human resource, the health examination of the human resource, executing engagement surveys for the human resource, executing stress checks for the human resource. Each index is associated based on the two axes including the axis for classifying indices into physical conditions and mental conditions and the axis for classifying indices into business management conditions and individual conditions. According to the job performance support apparatus of the second aspect, it is possible to clearly understand the current situation of each of the labor management of the human resource, the health examination of the human resource, the engagement survey of the human resource, and the stress check of the human resource (the above indices are the first indices) and the job performance result that is the second index. It is also possible to understand the association between the first indices and the second index for each of the four items of the first indices.

As for the effects of use of the index data, for example, labor management data is useful for maximizing the time efficiency of the human resource of the organization. Health examination data and stress check data can reduce waste and loss of business by maintaining the health condition of the human resource in a good condition. Engagement data is useful for realizing a workplace friendly for employees and leads to a high efficiency of works.

In the job performance support apparatus according to the third aspect of the present disclosure, the evaluation value is set in accordance with a determination item corresponding to each index, and the calculation unit evaluates the index data based on the determination item and calculates the evaluation value.

In the above-described configuration, in correspondence with the contents of each of the first indices and the second index, a determination item is set in correspondence with the pattern of evaluation value. Hence, when the determination items are referred to in accordance with the contents of each index in the department, and a corresponding determination item exists, an evaluation value corresponding to the determination item is added. There are, for example, five patterns of evaluation values. Five determination items may be set in accordance with the patterns, or determination items less than five or more than five may be set depending on the item. If determination items less than five are set, the number of patterns of evaluation values may be decreased in accordance with the number of determination items, or a determination item may correspond to two or more evaluation values. The acquired index data is evaluated based on the determination item for each index, and an evaluation value is calculated for each index.

Hence, according to the third aspect, it is possible to calculate the evaluation value in accordance with the determination item for each index and analyze the evaluation of the plurality of indices.

In the job performance support apparatus according to the fourth aspect of the present disclosure, a determination criterion of the evaluation value is set for each determination item, and the calculation unit calculates the evaluation value by comparing the index data with the determination criterion.

In the above-described configuration, since the determination criterion of the evaluation value is set for each determination item, the calculation unit can calculate the evaluation value by comparing the acquired index data with the determination criterion. The index data includes index contents of each index, and a determination criterion is set in correspondence with the index contents of each index. Hence, the determination criterion and the index contents are compared for each of all indices, and the evaluation value of the index contents is decided depending on which determination criterion is satisfied by the index contents.

In the job performance support apparatus according to the fifth aspect of the present disclosure, the first generation unit designates the display mode such that a graph to which a color corresponding to the evaluation value is added for each index is arranged at a position set for each index.

In the above-described configuration, since the first generation unit adds a color in correspondence with the evaluation value calculated for each index, it is possible to immediately understand which index is in a good condition and which index is in a bad condition at a glance. In addition, the color corresponding to the evaluation value is added to a graph arranged at a position set on an index basis. For this reason, when comparison is performed in each department, how the evaluation value of each index changes depending on the department can be understood at a glance.

In the job performance support apparatus according to the sixth aspect of the present disclosure, the first generation unit sets, based on characteristic data representing a characteristic of each department, a composition ratio of each index of the first indices to all the first indices in correspondence with the department, and designates the display mode of the first index based on the composition ratio.

In the above-described configuration, the first generation unit changes the display mode to display the evaluation values of the first indices based on the characteristic of each department. The characteristic is a property that characterizes the department and, more specifically, corresponds to a business target, how to progress with works, and the result of works. Hence, the characteristic generally changes between departments, and the index that is important for the improvement of job performance is different depending on the department. For this reason, the first generation unit sets a large composition ratio for an index that largely contributes to improvement of job performance in all the indices depending on the department. Also, in this aspect, the display form of the first indices is designated based on the composition ratio, thereby reflecting the composition ratio on the display form. For example, the first generation unit increases the display area by the index data of an index in proportion to the composition ratio. As a result, it is possible to immediately visually confirm the ratio of an index linked with job performance for the department, and it is therefore possible to grasp whether the work of the department is successful as an organization for high job performance. Also, based on the ratio of the index and the evaluation value added to the index, which index needs support can immediately be determined.

In the job performance support apparatus according to the seventh aspect of the present disclosure, the first generation unit sets an area to display the display mode corresponding to the evaluation value of the second index in accordance with the evaluation value.

In the above-described configuration, since the first generation unit increases the area of display corresponding to the evaluation value of the second index in proportion of the evaluation value, whether the job performance of the department is good can be known at a glance. In addition, when whether the job performance has exceeded the target value is defined by another element (for example, a color), the level of good job performance or the level of bad job performance can be known at a glance based on the area corresponding to the second index and the element. Hence, according to the seventh aspect, without taking time and labor for analysis on a department basis, it is possible to properly and quickly share information among the members in a meeting or the like or share an analysis result among the departments, and give many profits to management decisions and management responses.

In the job performance support apparatus according to the eight aspect of the present disclosure, the second generation unit sets training data including training contents corresponding to each of the first indices as the support measure based on the combination of the display data.

In the above-described configuration, the number of combinations of display data representing the evaluation values of the indices is decided by the number of values that an evaluation value can take and the number of indices. For example, if an evaluation value can take three values, and the number of indices is five, the number of combinations is 3⁵ = 243. In fact, these combinations include combinations with almost the same support measure and combinations that are hardly implemented. Hence, in many cases, setting support measures only in combinations of several patterns suffices. An actual combination pattern may be set by trying several patterns.

Since at least the outline of a support measure is decided by the combination of display data, a training corresponding to each first index is set based on the combination of display data. Hence, according to the eighth aspect, in accordance with the combination of display data of indices, training data to be provided to the human resource of a department is provided as an individual support measure for each first index. It is possible to provide a finer training corresponding to the contents of the first indices for each department. As a result, it is possible to easily analyze and understand what kind of training should be executed in each department to improve a first index and influence the job performance improvement of the department.

The job performance support apparatus according to the ninth aspect of the present disclosure further comprises a presentation unit configured to present the display data and the support data according to the combination.

In the above-described configuration, the job performance support apparatus further includes the presentation unit, and displays display data and support data to a manager or the like of the organization. Hence, according to the ninth aspect, the manager or the like and the user who uses the job performance support apparatus can simultaneously refer to the display data and the support data. In addition, the presentation unit may be provided in a terminal apparatus or the like different from the job performance support apparatus, and setting may be done such that these data are referred to on the terminal apparatus or the like.

In the job performance support apparatus according to the 10th aspect of the present disclosure, the presentation unit further presents associated data associated with the display data of each index.

In the above-described configuration, the associated data further presented by the presentation unit can be any data if it is associated with display data. For example, the associated data is data as the base of index contents corresponding to display data. Examples of the associated data are attendance data of labor management, result data of a health examination, result data of an engagement survey, or result data of a stress check. The presentation unit presents data as the base of display data. Hence, according to the 10th aspect, since the presentation unit can present various associated data associated with display data, the manager or the like of a department can examine detailed data of a human resource and use the data for a measure in the future.

According to the present invention, it is possible to provide a job performance support apparatus configured to provide information for improving the job performance of an organization and support a member of the organization, a method, and a program.

### BRIEF DESCRIPTION OF THE DRAWINGS

FIG. 1 is a view showing the outline of a system including a job performance support apparatus (server) according to the embodiment, a network, and a terminal apparatus connected to the server via the network;
FIG. 2 is a view schematically showing an example of the hardware configuration of the job performance support apparatus according to the embodiment;
FIG. 3 is a view showing an example of a part of the software configuration of the job performance support apparatus according to the embodiment;
FIG. 4 is a view schematically showing an example of the hardware configuration of the terminal apparatus according to the embodiment;
FIG. 5 is a view showing an example of a part of the software configuration of the terminal apparatus according to the embodiment;
FIG. 6 is a flowchart schematically showing an example of a processing procedure concerning the job performance support apparatus according to the embodiment;
FIG. 7 is a flowchart schematically showing an example of a processing procedure concerning the terminal apparatus according to the embodiment;
FIG. 8 is a table that collects, for an example of a plurality of indices, purposes and things found from the indices;
FIG. 9 is a table showing categories and explanations concerning the contents of an engagement survey;
FIG. 10 is a view showing the difference between a stress check and an engagement survey;
FIG. 11 is a view showing an example of associated data serving as the base of evaluation of labor management that is one of the indices;
FIG. 12 is a view showing an example of associated data serving as the base of evaluation of a stress check that is one of the indices;
FIG. 13A is a view showing an example of associated data serving as the base of evaluation of a health examination that is one of the indices;
FIG. 13B is a view showing an example of associated data following FIG. 13A;
FIG. 14 is a view showing an example of associated data serving as the base of evaluation of an engagement survey that is one of the indices;
FIG. 15 is a flowchart showing an example of evaluation value calculation processing of step S603 in FIG. 6;
FIG. 16 is a table showing an example of association between determination criteria, score values, and colors of job performance that is one of the indices;
FIG. 17 is a table showing an example of association between determination criteria, score values, and colors of the health examination that is one of the indices;
FIG. 18 is a table showing an example of association between determination criteria, score values, and colors of the labor management that is one of the indices;
FIG. 19 is a table showing an example of association between determination criteria, score values, and colors of the stress check that is one of the indices;
FIG. 20 is a table showing an example of association between determination criteria, score values, and colors of the engagement survey that is one of the indices;
FIG. 21 is a flowchart showing an example of display form setting processing of step S604 in FIG. 6;
FIG. 22 is a graph showing an example of a display form in which evaluation values obtained by evaluating five indices are displayed in colors;
FIG. 23 shows graphs showing an example in a case in which the composition ratio of indices is set for each department concerning four indices (first indices);
FIG. 24 shows graphs showing an example in a case in which concerning an index (second index) representing job performance, the display area is set in accordance with the evaluation value of job performance;
FIG. 25 is a flowchart showing an example of support data generation processing of step S606 in FIG. 6;
FIG. 26 is a view showing an example of the correspondence between a problem category to which each department belongs and a support measure by combining the display data of the five indices;
FIG. 27 is a view for explaining an example of training concerning the labor management that is one of the first indices;
FIG. 28 is a view for explaining an example of training concerning the health examination that is one of the first indices;
FIG. 29 is a view for explaining an example of training concerning the engagement survey that is one of the first indices;
FIG. 30 is a view for explaining an example of training concerning the stress check that is one of the first indices; and
FIG. 31 is a view for explaining an example of training concerning the stress check that is one of the first indices.

### DETAILED DESCRIPTION

An embodiment (to be also referred to as "this embodiment" hereinafter) according to one aspect of the present invention will now be described with reference to the accompanying drawings. Note that in the following embodiment, parts denoted the same reference numerals perform similar operations, and a repetitive description thereof will be omitted.

### [Outline]

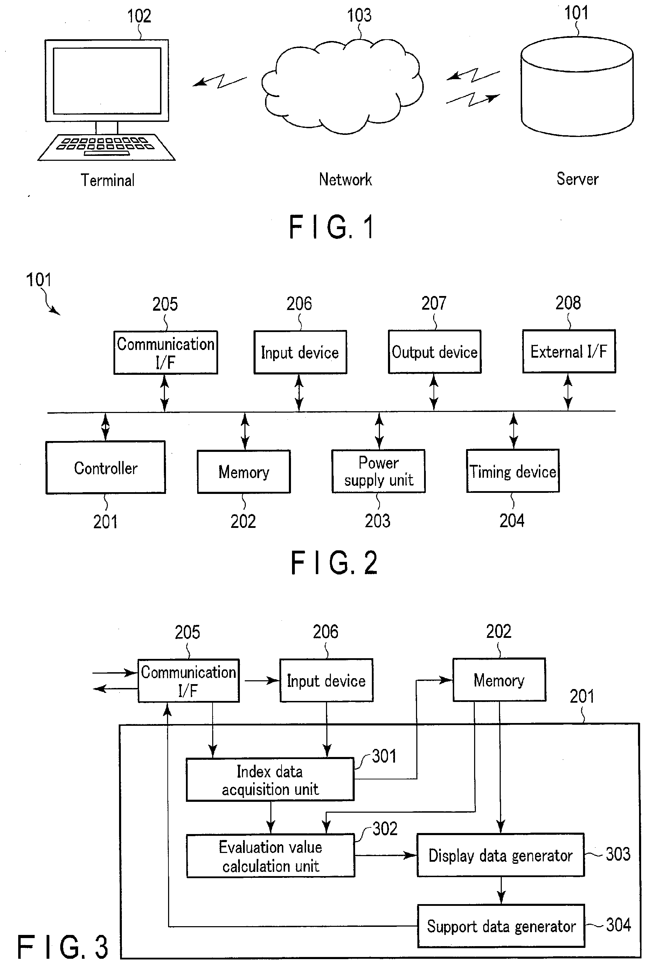

The outline of a job performance support apparatus according to the present invention will be described first with reference to FIG. 1. FIG. 1 schematically shows a job performance support apparatus (typically, a server) 101 according to an example of the outline, a terminal apparatus 102, and a network 103.

The job performance support apparatus 101 is, for example, a server, receives data concerning business via the network 103, and creates, for each department of an organization, display data in which the display mode of an evaluation value is designated for each index, and the evaluation value is associated with the index, and support data decided based on the display data.

Data concerning business are mainly provided from a personnel department or the like in a company. The data concerning business are index data including index contents corresponding to a plurality of first indices classified by at least two axes including an axis for classifying the indices into physical conditions and mental conditions and an axis for classifying indices into business management conditions and individual conditions in the organization, and a second index concerning the job performance result of the department. The index data of the first indices are, for example, labor management data, health examination data, engagement data, and stress check data. The index data of the second index is job performance data.

It is also considered that the four first indices are obtained by roughly classifying the first indices into physical conditions and mental conditions and then classifying these into organization and/or business conditions and individual conditions.

The display data is data generated for each index data by calculating an evaluation value for each index from acquired index data and designating a display mode representing a form to display the evaluation value and used to display the evaluation value in the display mode.

The support data is generated in association with the combination of display data for each index, and includes a support measure corresponding to a first index. The support data includes a support measure for a department included in an organization.

The terminal apparatus 102 is configured to acquire display data and/or support data from the job performance support apparatus 101 via the network 103 and display the data. The terminal apparatus 102 can be any device capable of displaying these data and is, for example, a personal computer, a laptop, a smartphone, a smartwatch, or a tablet.

As described above, according to the job performance support apparatus 101 of this embodiment, the current situation in a department of an organization can be analyzed, together with the job performance of the department, by at least two axes including the axis for classifying indices into physical conditions and mental conditions of a human resource and the axis for classifying indices into business management conditions and individual conditions. Also, according to this embodiment, it is possible to clearly understand, for each department, how the plurality of indices are related to job performance. Since not only the understanding but also clearly showing a problem of a department and presenting a support measure for the problem is possible, it is possible to execute a detailed and appropriate measure for each personnel of a department.

### [Configuration Examples]

### (Hardware Configuration)

### <Job Performance Support Apparatus 101>

An example of the hardware configuration of the job performance support apparatus 101 according to this embodiment will be described next with reference to FIG. 2.

As shown in FIG. 2, the job performance support apparatus 101 according to this embodiment includes a computer in which a controller 201, a memory 202, a power supply unit 203, a timing device 204, a communication interface 205, an input device 206, an output device 207, and an external interface 208 are electrically connected. The job performance support apparatus 101 according to this embodiment corresponds to "job performance support apparatus" in the present invention. Note that in FIG. 2, the communication interface and the external interface are shown as "communication I/F" and "external I/F", respectively.

The controller 201 includes a CPU (Central Processing Unit), a RAM (Random Access Memory), and/or a ROM (Read Only Memory), and controls each constituent element in accordance with information processing. The controller 201 executes a program configured to calculate an evaluation value for each index from acquired index data, generate, for each index, display data in which the display mode of an evaluation value is designated for each index, and the evaluation value is associated with the index, and generate, for each department, support data including support measures corresponding to the first indices in accordance with the combination of the display data of the indices. The program is stored in the memory 202, and the controller 201 reads out the execution program from the memory 202 and executes processing.

The memory 202 is a medium configured to store information such as a program by an electrical, magnetic, optical, mechanical, or chemical action such that a computer or another device, or a machine or the like can read the recorded information such as a program. The memory 202 is, for example, an auxiliary storage device such as a hard disk drive or a solid-state drive, and stores an execution program configured to generate, for each index, display data in which an evaluation value is associated with each index, and the display mode of the evaluation value changes between the indices based on index data corresponding to one or more indices and generate, for each department, support data including support measures corresponding to the first indices in accordance with the combination of the display data.

In addition, the memory 202 stores display data and support data generated by the program executed by the controller 201. Also, the memory 202 stores index data acquired by the communication interface 205 and/or the input device 206. Furthermore, the memory 202 may store associated data associated with display data for each index.

The memory 202 also stores determination criterion data including determination criteria used by the program to determine the evaluation value for each index when generating display data. Also, the memory 202 stores characteristic data that describes the characteristic of each department of the organization. The characteristic data is input by the input device 206 or input from an external device via the communication interface 205. The characteristic data sets, for each department, association (for example, an association degree) between each index of the plurality of first indices and the second index (that is, job performance) based on the main business target, how to progress with works, the result of works, and the like in the department.

Furthermore, the memory 202 may store problem categories corresponding to the combination of display data of indices in advance. A problem category indicates whether the department has a problem, and if a problem exists, what kind of problem exists. More specifically, the problem categories include, for example, soundness, labor measure, mental, and business risk. Soundness indicates a state without a problem, and a measure for maintaining this state is necessary. Labor measure corresponds to a case in which the evaluation of an index of labor management is low. Mental corresponds to a case in which the evaluation of an index of engagement surveys and stress checks corresponding to mental conditions is low. Business risk corresponds to a case in which the evaluations of all the first indices are low.

The memory 202 may also include a drive. The drive is a device configured to accept stored data from an auxiliary storage device, a recording medium, or the like and, more particularly, load a program, and is, for example, a semiconductor memory drive (flash memory drive), a CD (Compact Disk) drive, or a DVD (Digital Versatile Disk) drive. The type of the drive may appropriately be selected in accordance with the type of the storage medium. Data and the like acquired from the above-described execution program and/or the network 103 may be stored in the storage medium.

The power supply unit 203 can be any unit capable of supplying power to device parts included in the job performance support apparatus 101, and is, for example, a chargeable secondary battery or an AC power supply that can be acquired from a normal electric outlet. The power supply unit 203 supplies power to each element mounted in the main body of the job performance support apparatus 101. The power supply unit 203 supplies power to, for example, the controller 201, the memory 202, the timing device 204, the communication interface 205, the input device 206, the output device 207, and the external interface 208.

The timing device 204 is a device configured to measure time and can measure a date/time. For example, the timing device 204 is a clock including a calendar and transfers the information of the current year and month and/or date/time to the controller 201. The timing device 204 is used to, for example, add a date/time when the controller 201 generates display data and support data.

The communication interface 205 is, for example, a short distance wireless communication (for example, Bluetooth^{®}) module, a wired LAN (Local Area Network) module, a wireless LAN module, or the like, and is an interface configured to perform wired or wireless communication via the network. The communication interface 205 is an interface configured to connect the job performance support apparatus 101 to an external device (for example, a computer, a server, or a communication device on the network). The communication interface 205 is controlled by the controller 201 and receives index data from another apparatus such as a server and/or another terminal apparatus via the network 103, and/or transmits display data and support data generated by the job performance support apparatus 101 to the terminal apparatus 102 (for example, a smartphone and/or a computer) or the like via the network 103. In addition, the communication interface 205 may be configured to download a program to be executed by the job performance support apparatus 101 from a specific server (not shown) in which the program is stored in advance or upload the program to the terminal apparatus 102. When the terminal apparatus 102 receives the program, the program is executed by the terminal apparatus 102 to generate display data and support data, and these data are presented by the terminal apparatus 102.

Communication via the network 103 may be either wireless or wired. Note that the network 103 may be an internetwork including the Internet, a network of another type such as an intra-hospital LAN, or one-to-one communication using a USB (Universal Serial Bus) cable or the like. The communication interface 205 may include a micro USB connector.

The input device 206 is a device configured to accept an input and is, for example, a touch panel, a physical button, a mouse, a keyboard, or the like. The output device 207 is a device configured to perform output and output information by display, a voice, or the like and, is, for example, a display, a loudspeaker, or the like. Index data may be input by the input device 206.

The external interface 208 mediates between the main body and the job performance support apparatus 101 and an external device, and is, for example, a USB port or an interface used to connect an external device (for example, a printer, a memory, or a communication device).

### (Software Configuration)

### <Job Performance Support Apparatus 101>

An example of the software configuration of the job performance support apparatus 101 according to this embodiment will be described next with reference to FIG. 3. FIG. 3 shows a software configuration for executing a program executed by the controller 201 of the job performance support apparatus 101 and configured to calculate an evaluation value for each index from acquired index data, generate, for each index, display data in which the display mode of an evaluation value is designated for each index, and the evaluation value is associated with the index, and generate, for each department, support data including support measures corresponding to the first indices in accordance with the combination of the display data of the indices.

When executing a necessary program, the controller 201 of the job performance support apparatus 101 expands, in the RAM, an execution program configured to generate display data for each index based on data stored in the memory 202, which is acquired from another server and/or another terminal apparatus via the network 103, and generate, for each department, support data including support measures corresponding to the first indices in accordance with the combination of display data. The controller 201 causes the CPU to interpret and execute the execution program expanded in the RAM and used to generate support data for each department, thereby controlling each constituent element. As shown in FIG. 3, the job performance support apparatus 101 according to this embodiment includes an index data acquisition unit 301, an evaluation value calculation unit 302, a display data generator 303, and a support data generator 304.

The index data acquisition unit 301 acquires index data of each department from the communication interface 205 and/or the input device 206, and stores the index data of each department in the memory 202. In addition, the index data acquisition unit 301 transfers the index data of each department to the evaluation value calculation unit 302. Also, the index data acquisition unit 301 acquires characteristic data representing the characteristic of each department of an organization, and display designation data (that is, data that designates a display mode) that designates the type of a graph for displaying display data, a position to arrange the evaluation value of each index, and a color corresponding to the evaluation value of each index from the communication interface 205 and/or the input device 206 and transfers the data to the memory 202.

The evaluation value calculation unit 302 receives the index data of each department from the index data acquisition unit 301, and loads, from the memory 202, determination criterion data including determination criteria to be used to determine a score value (also called an evaluation value) of each index. The evaluation value calculation unit 302 compares each index with corresponding determination criteria, and calculates a score value corresponding to each index. Determination criteria are set in association with score values. If certain determination criteria are satisfied, a corresponding score value is decided. For the determination criteria, five score values are set, and the score values are set stepwise from a score value "1" representing soundness to a score value "5" representing a warning. The evaluation value calculation unit 302 transfers, to the display data generator 303, an evaluation value calculated for each index for one or more departments.

The display data generator 303 acquires, from the memory 202, display designation data of each department for each index for one or more departments, and generates, for each of the first indices and the second index, display data in which an evaluation value acquired from the evaluation value calculation unit 302 is associated with a display mode by a corresponding display mode. As for the second index, the display data generator 303 decides the area of a graph used for display in accordance with the evaluation value, and generates the display data of the second index including the area data.

The larger the job performance result is, the larger the display data generator 303 sets the area of the graph corresponding to the second index. For example, the area may be changed in accordance with the target achievement of job performance (or the contribution to the job performance of a whole organization). A standard value may be set for the area of the graph corresponding to the second index, and the area may be set in proportion to the achievement in correspondence with the graph having the area for a target achievement of 100% as a standard value.

The support data generator 304 acquires display data for each index for one or more departments, selects a problem category corresponding to the combination of display data of indices, and generates, for each department, support data including a support measure corresponding to the category. The generated support data is transferred from the support data generator 304 to the communication interface 205 and transferred from the communication interface 205 to the terminal apparatus 102.

When the support data generator 304 selects the problem category, a table in which combinations of display data and problem categories are set may be stored in the memory 202 in advance, and the support data generator 304 may refer to the table and select a problem category corresponding to a combination of display data.

### <Others>

The operation of the job performance support apparatus 101 will be described in detail using an operation example to be described later. Note that in this embodiment, the controller 201 of the job performance support apparatus 101 may be implemented by a general-purpose CPU. However, some or all of the above-described operations (or functions) may be implemented by one or a plurality of dedicated processors. Also, concerning the configuration of the job performance support apparatus 101, omission, replacement, and addition may be done as needed in accordance with the embodiment.

### [Configuration Examples]

### (Hardware Configuration)

### <Terminal Apparatus 102>

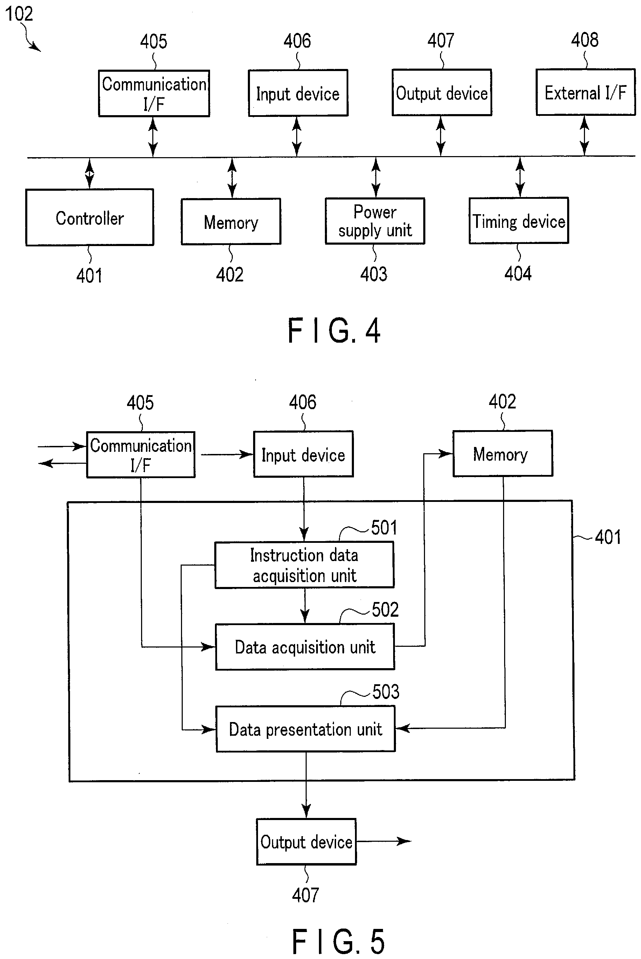

An example of the hardware configuration of the terminal apparatus 102 according to this embodiment will be described next with reference to FIG. 4.

As shown in FIG. 4, the terminal apparatus 102 according to this embodiment includes a computer in which a controller 401, a memory 402, a power supply unit 403, a timing device 404, a communication interface 405, an input device 406, an output device 407, and an external interface 408 are electrically connected. Note that in FIG. 4, the communication interface and the external interface are shown as "communication I/F" and "external I/F", respectively.

Since the device parts shown in FIG. 4 perform operations similar to those of the device parts with the same names (but different reference numerals) shown in FIG. 2, a detailed description thereof will be omitted, and only different points will be described.

The controller 401 executes a program configured to present display data, support data, associated data, and the like transmitted from the job performance support apparatus 101.

The memory 402 stores programs to be executed by the controller 401 and data transmitted from the job performance support apparatus 101.

If data is received from the job performance support apparatus 101, the timing device 404 records a date/time if an instruction for presenting specific data is received from the input device 406.

### (Software Configuration)

### <Terminal Apparatus 102>

An example of the software configuration of the terminal apparatus 102 according to this embodiment will be described next with reference to FIG. 5. FIG. 5 shows a software configuration for executing a program to present display data, support data, associated data, and the like generated by the job performance support apparatus 101.

An instruction data acquisition unit 501 acquires instruction data from the input device 406, and transfers the instruction data to the data acquisition unit 502 or the data presentation unit 503 by the instruction data. As the instruction data, there are data including an instruction for acquiring desired data from the job performance support apparatus 101, and data including an instruction for presenting one of data stored in the memory 402. If the input device 406 instructs to present predetermined data, but the data is not stored in the memory 402, the instruction data acquisition unit 501 may acquire the data from the job performance support apparatus 101 and present the data.

If instruction data for acquiring desired data is received from the instruction data acquisition unit 501, the data acquisition unit 502 acquires the desired data from the job performance support apparatus 101 via the communication interface 405, and stores the acquired data in the memory 402.

If instruction data for presenting data is received from the instruction data acquisition unit 501, the data presentation unit 503 acquires the data designated by the instruction data from the memory 402, and causes the output device 407 to present the data.

### <Others>

The operation of the terminal apparatus 102 will briefly be described using an operation example to be described later. Note that in this embodiment, the controller 401 of the terminal apparatus 102 may be implemented by a general-purpose CPU. However, some or all of the above-described operations (or functions) may be implemented by one or a plurality of dedicated processors. Also, concerning the configuration of the terminal apparatus 102, omission, replacement, and addition may be done as needed in accordance with the embodiment.

### [Operation Example: Job Performance Support Apparatus 101]

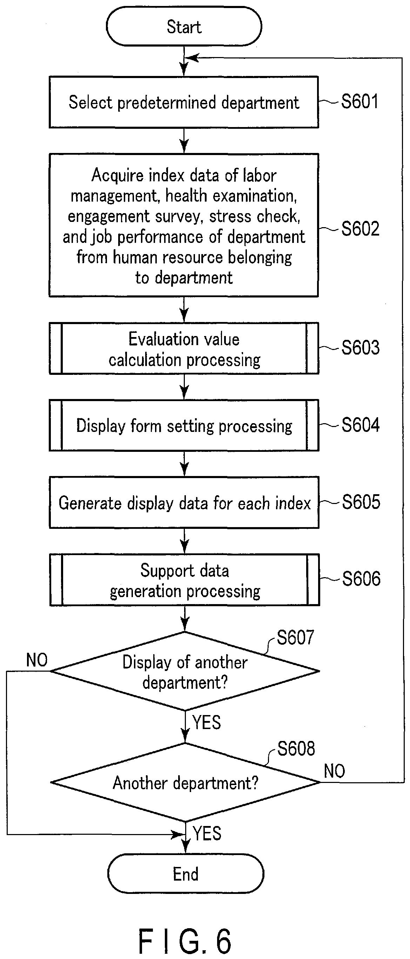

An example of a processing procedure of generating display data and support data of an organization by the job performance support apparatus 101 will be described next with reference to FIG. 6.

FIG. 6 is a flowchart showing an example of a processing procedure of the job performance support apparatus 101. Note that the processing procedure to be described below is merely an example, and each process may be changed as much as possible. Also, concerning the processing procedure to be described below, omission, replacement, and addition of steps can be done as needed in accordance with the embodiment.

### (Activation)

First, a user or a manager who can access the job performance support apparatus 101 activates the job performance support apparatus 101 via the input device 206, and an input from an organization as the target of job performance support may also be accepted. A description will be made here assuming that the organization is determined in advance. The controller 201 of the job performance support apparatus 101 performs processing in accordance with the following processing procedure.

### (Step S601)

In step S601, the controller 201 operates as the index data acquisition unit 301. The index data acquisition unit 301 selects a department from departments included in the organization. The department selection is normally done in accordance with a predetermined rule. However, a department may be selected in accordance with an instruction from the input device 206. The predetermined rule is not particularly limited, and for example, selection is done at random, in the Japanese syllabary order, or in the alphabetical order.

### (Step S602)

In step S602, the controller 201 operates as the index data acquisition unit 301, and acquires index data including index contents corresponding to a plurality of first indices and a second index from a server or the like in the organization via the communication interface 205.

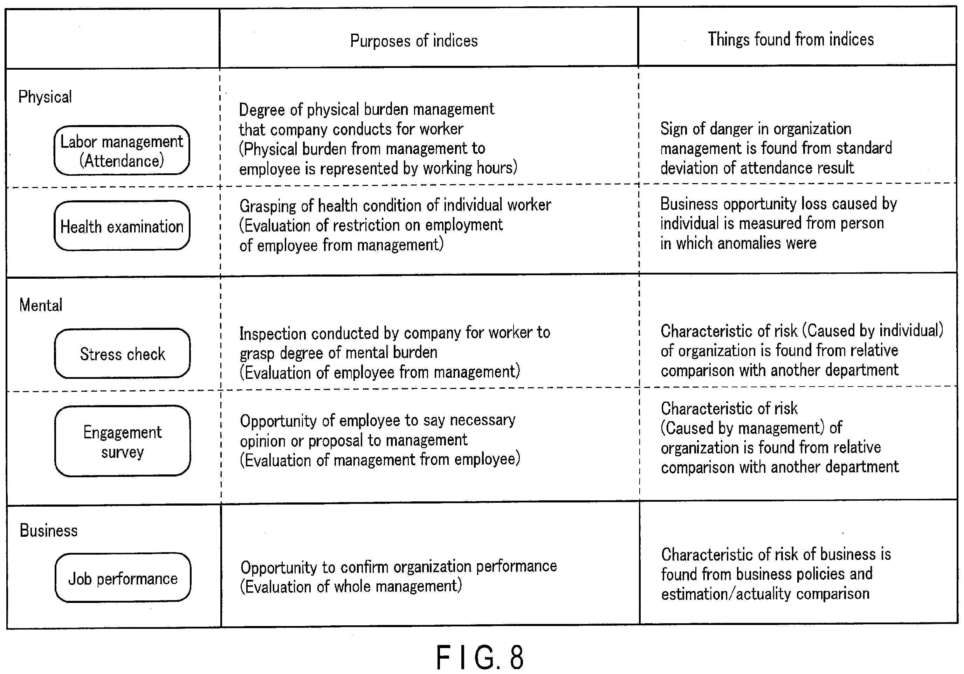

As shown in FIG. 8, the first indices include labor management and a health examination classified into physical conditions, and also include a stress check and an engagement survey classified into mental conditions. The second index is a business. Examples of the purposes of the indices and things found from the index contents corresponding to the indices are shown in the table of FIG. 8. Indices derived from individuals are the health examination and the stress check in the above-described indices, and indices derived from the management or business are the labor management and the engagement survey in the above-described indices.

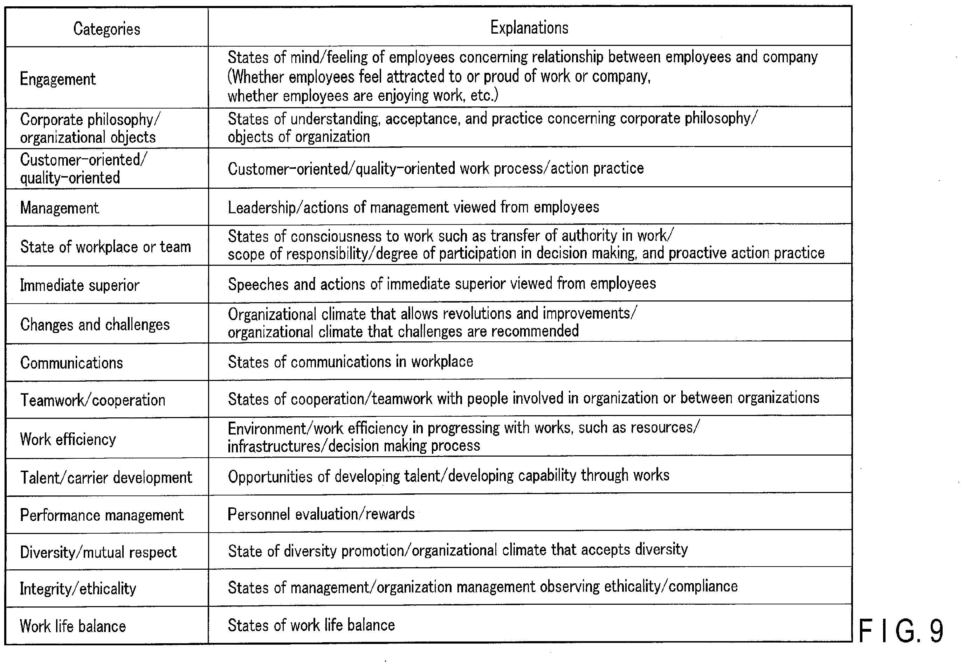

Categories for the engagement survey and an explanation thereof are shown in the table of FIG. 9. The contents of the engagement survey are created based on, for example, answers collected from the members of the organization concerning a questionnaire corresponding to the contents of the category to investigate the contents in the category. The distributions of the answers are compared between departments, thereby finding the characteristic of the risk of the organization.

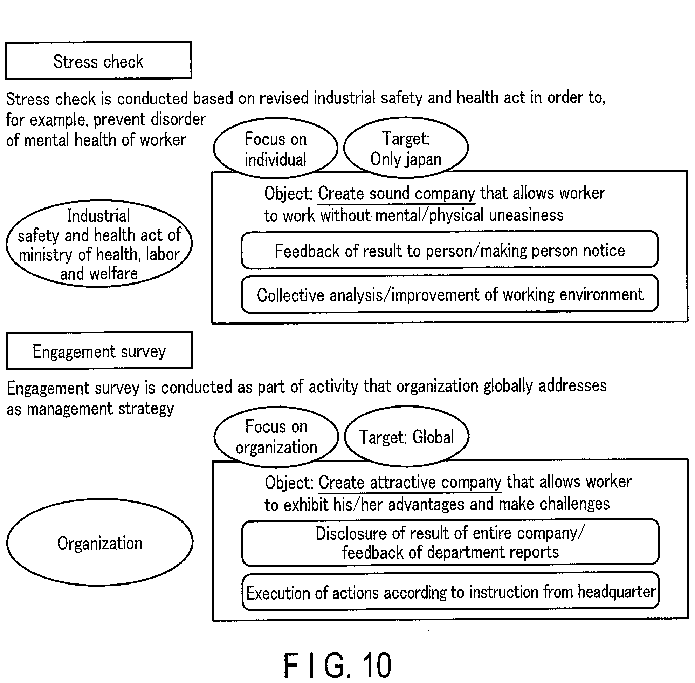

FIG. 10 shows an explanation of the difference between the stress check and the engagement survey. As shown in FIG. 10, the stress check aims at preventing the mental health of an individual worker from becoming bad and allowing him/her to work in the organization without uneasiness. The engagement survey aims at executing the management strategy of the organization, which is derived from management, in the whole organization and forming an attractive and meaningful organization.

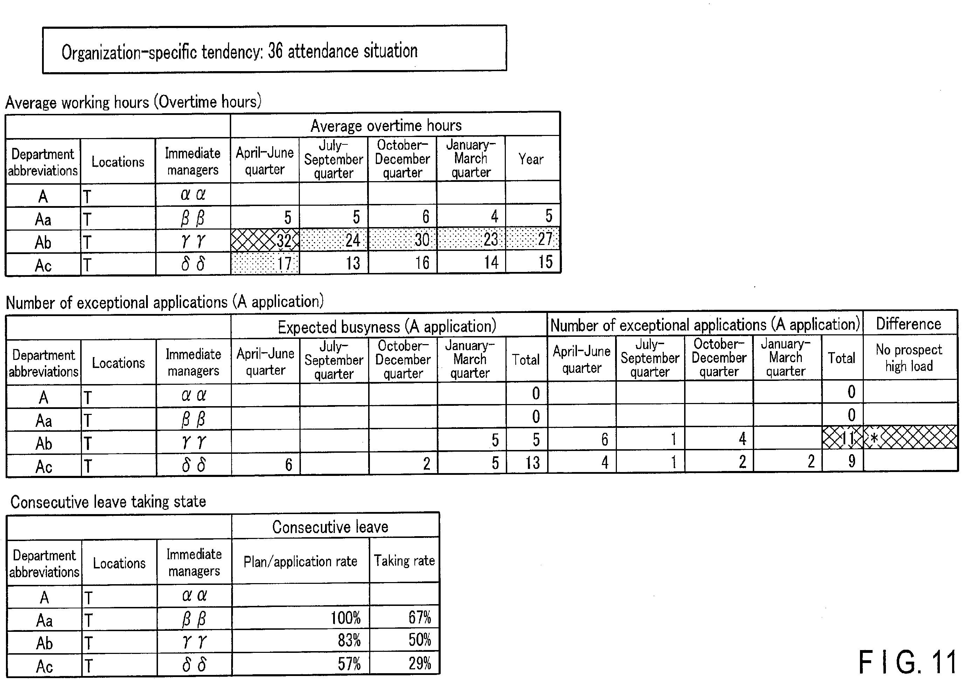

FIGS. 11, 12, 13A, 13B, and 14 show examples of data concerning the index contents of the first indices. These data are examples of associated data serving as the base of evaluation in the labor management, the stress check, the health examination, and the engagement survey. Note that these data can also be associated data of corresponding display data.

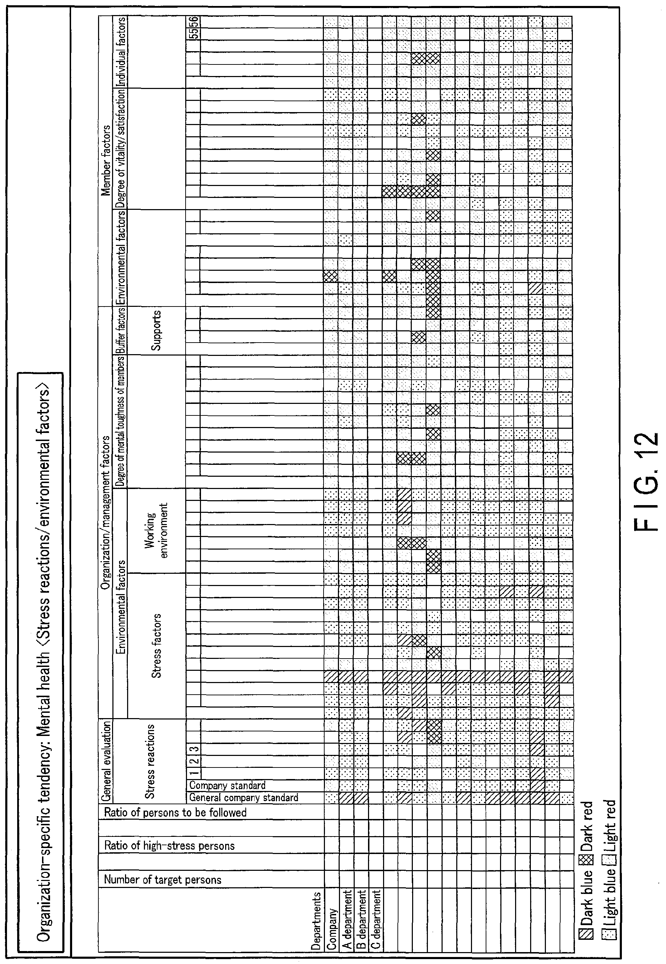

FIG. 11 shows data including the attendance situation in each department including detailed personal information. FIG. 12 shows the result of totaling questionnaire results concerning a mental health for each department in correspondence with various factors. In the example shown in FIG. 12, there are questions 1 to 56, and a suggestion concerning a specific state of mental health can be obtained based on the answers. In FIG. 12, if a good state is assumed for each factor in each department, the state is indicated by blue, and if a bad state is assumed, the state is indicated by red for each factor. In FIG. 12, light blue indicates a good state whose degree is slightly lower than in deep blue, and light red indicates a bad state whose degree is slightly lower than in deep red.

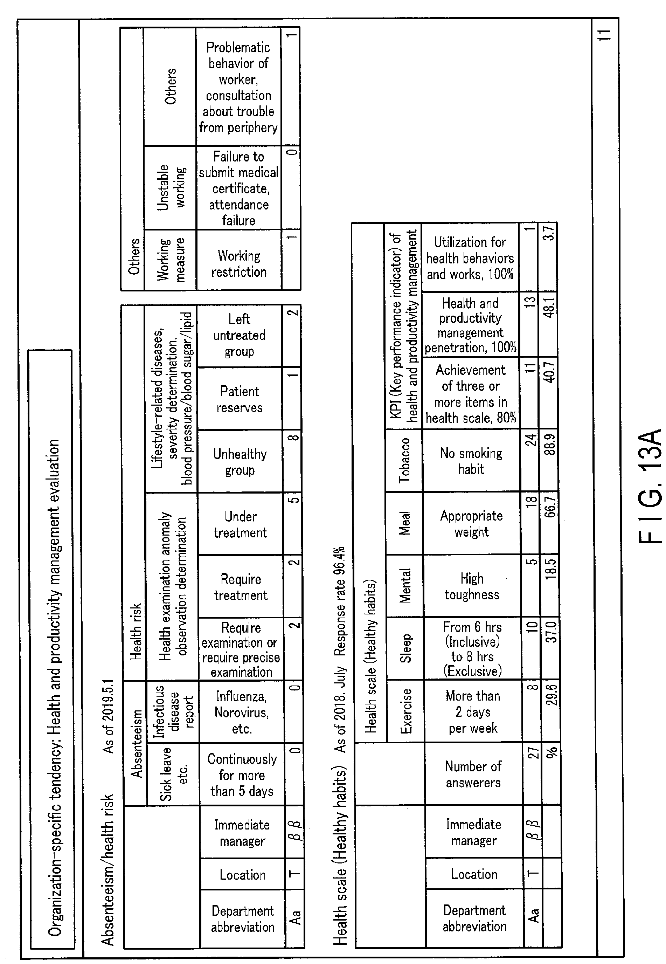

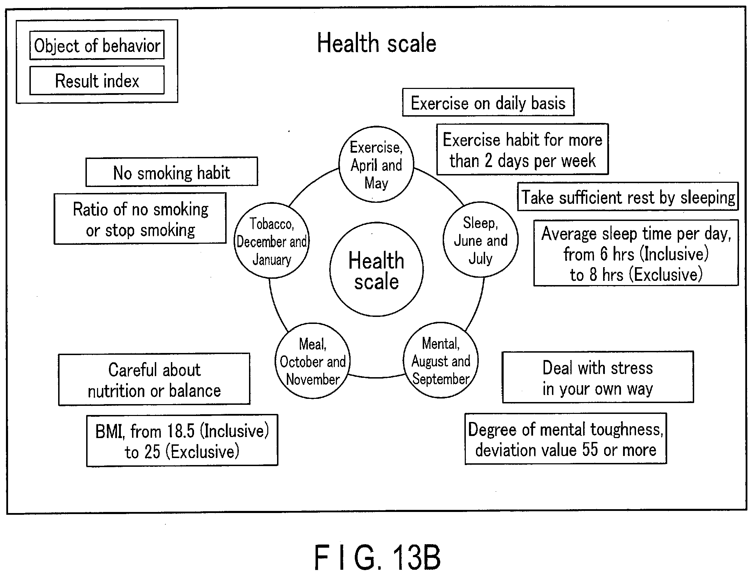

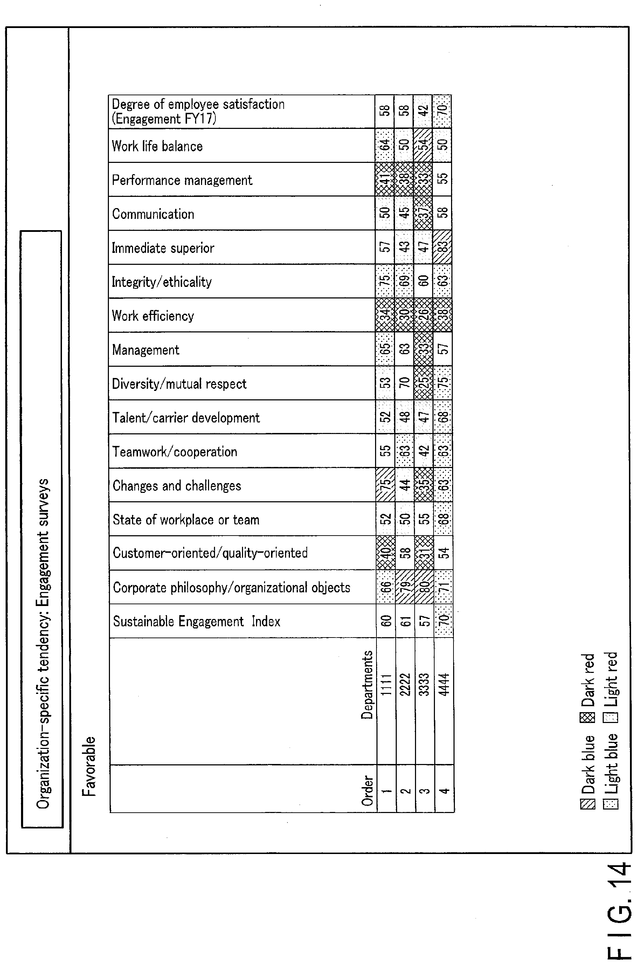

FIGS. 13A and 13B show data concerning health and productivity management and health examinations. Absenteeism shown in FIG. 13A indicates a state of absence (particularly, long-term medical treatment) caused by a disease or a poor physical condition. A similar concept is presenteeism (this item may be included in the data shown in FIG. 13A), which indicates a state in which a person is working despite being in a state in which he/she preferably takes a rest because of a disease or a poor physical condition. FIG. 14 shows the result of totaling questionnaire results concerning an engagement survey for each department in correspondence with various factors. Colors in FIG. 14 have the same meanings as in FIG. 12.

### (Step S603)

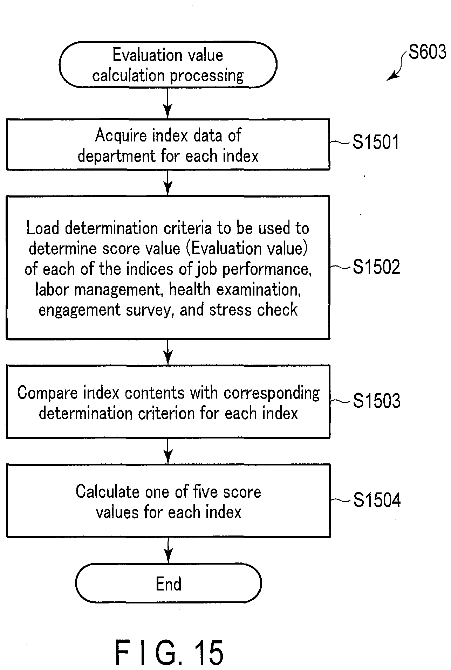

In step S603, the controller 201 operates as the evaluation value calculation unit 302. The evaluation value calculation unit 302 receives the index data of the department selected in step S601 from the index data acquisition unit 301, and calculates a score value for each index. A detailed procedure will be described later with reference to FIG. 15.

### (Step S604)

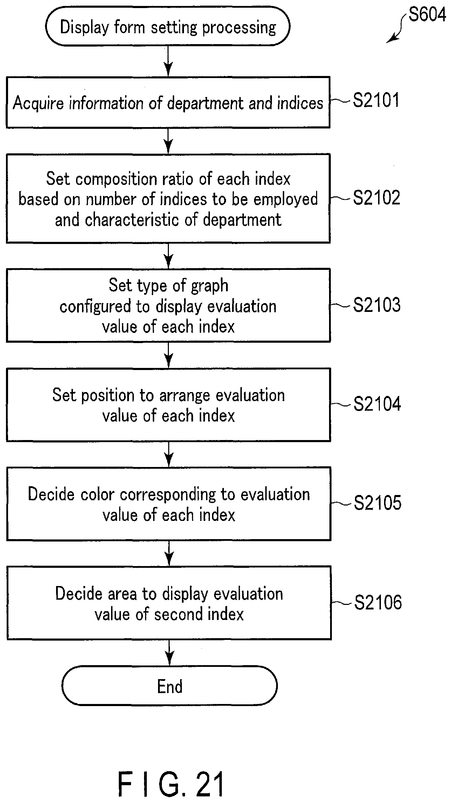

In step S604, the controller 201 operates as the display data generator 303. For the one or more departments, the display data generator 303 sets a display mode representing a form to display the evaluation values calculated for the first indices and the second index by the display designation data of the department for each index. A detailed procedure will be described later with reference to FIG. 21.

### (Step S605)

In step S605, the controller 201 operates as the display data generator 303 and, for the evaluation values acquired from the evaluation value calculation unit 302, generates display data for each of the first indices and the second index based on the display mode set in step S604.

### (Step S606)

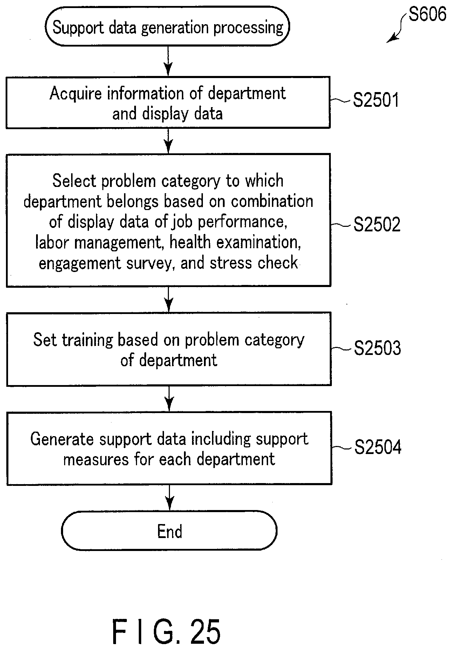

In step S606, the controller 201 operates as the support data generator 304, acquires the display data generated in step S605 for each index in the department, selects a problem category corresponding to the combination of the display data of the indices, and generates, for each department, support data including support measures corresponding to the first indices corresponding to the category. A detailed procedure will be described later with reference to FIG. 25.

### (Step S607)

In step S607, the controller 201 operates as the index data acquisition unit 301, and determines whether another department to be displayed exists. If another department to be displayed exists, the process advances to step S608. If another department to be displayed does not exist, the processing is ended.

### (Step S608)

In step S608, the controller 201 operates as the index data acquisition unit 301, and determines whether another department of the organization exists. Upon determining that another department exists, the process returns to step S601 to select one of the remaining departments and continue the processing from step S602.

### [Operation Example: Terminal Apparatus 102]

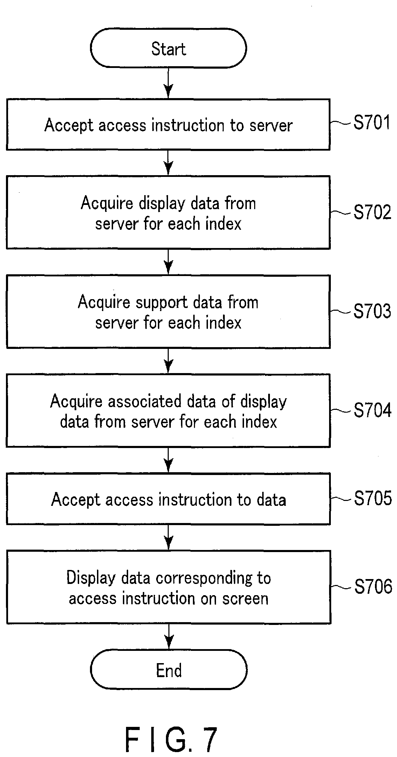

An example of a processing procedure of acquiring, by the terminal apparatus 102, display data and support data from the job performance support apparatus 101 (server) and presenting these data will be described next with reference to FIG. 7.

FIG. 7 is a flowchart showing an example of a processing procedure of the instruction data acquisition unit 501, the data acquisition unit 502, and the data presentation unit 503. Note that the processing procedure to be described below is merely an example, and each process may be changed as much as possible. Also, concerning the processing procedure to be described below, omission, replacement, and addition of steps can be done as needed in accordance with the embodiment.

### (Step S701)

In step S701, the controller 401 operates as the instruction data acquisition unit 501, and accepts an access instruction from the input device 406 to the job performance support apparatus 101. The access instruction is an instruction to, for example, display the display data, the support data, and/or the associated data of a certain department.

### (Step S702)

In step S702, the controller 401 operates as the data acquisition unit 502, and acquires corresponding data from the job performance support apparatus 101 based on the access instruction in step S701. In an example of the flowchart, the data acquisition unit 502 acquires display data from the job performance support apparatus 101 for each index. Also, the data acquisition unit 502 stores the acquired display data in the memory 402.

### (Step S703)

In step S703, the controller 401 operates as the data acquisition unit 502, and acquires corresponding support data for each display data acquired in step S702 on an index basis based on the access instruction in step S701. Also, the data acquisition unit 502 stores the acquired support data in the memory 402.

### (Step S704)

In step S704, the controller 401 operates as the data acquisition unit 502, and acquires corresponding associated data for each display data acquired in step S702 on an index basis based on the access instruction in step S701. Also, the data acquisition unit 502 stores the acquired associated data in the memory 402.

### (Step S705)

In step S705, the controller 401 operates as the data presentation unit 503, and accepts the access instruction in step S501.

### (Step S706)

In step S706, the controller 201 operates as the data presentation unit 503, and outputs data corresponding to the access instruction accepted in step S705 to the output device 407. The output device 407 presents the data to a user such as a manager of the organization or the department. The output device 407 presents the data to the user normally by displaying the data on a display, but the method is not particularly limited to the display. Any presentation method can be used if it appeals to the five senses of a human and makes the human to understand. For example, the data may be output by a voice, or may be output in braille.

### [Operation Example: Step S603, Evaluation Value Calculation Processing]

Processing of the evaluation value calculation unit 302 to acquire index data for all indices of a department and calculate a score value for each index will be described next with reference to FIG. 15.

FIG. 15 is a flowchart showing an example of the processing procedure of step S603. Note that the processing procedure to be described below is merely an example, and each process may be changed as much as possible. Also, concerning the processing procedure to be described below, omission, replacement, and addition of steps can be done as needed in accordance with the embodiment.

### (Step S1501)

In step S1501, the controller 201 operates as the evaluation value calculation unit 302 (in the description of FIG. 15, the main operation component is the same and will be omitted below), and acquires, from the index data acquisition unit 301, index data representing the index contents of each index of the department selected in step S601. Note that in this embodiment, as described concerning step S602, the indices include labor management, a health examination, a stress check, an engagement survey (these four indices are the first indices), and job performance (second index).

### (Step S1502)

In step S1502, the controller 201 loads, from the memory 202, determination criterion data including determination criteria to be used to determine a score value of each index from the index data acquired in step S1501.

### (Step S1503)

In step S1503, the controller 201 compares determination criteria corresponding to the index contents of each index from the determination criterion data loaded in step S1502 with the index contents of the index. For example, a plurality of determination criteria are set for each index, and a score value can be added to each index depending on which determination criteria are satisfied or whether all determination criteria are not satisfied.

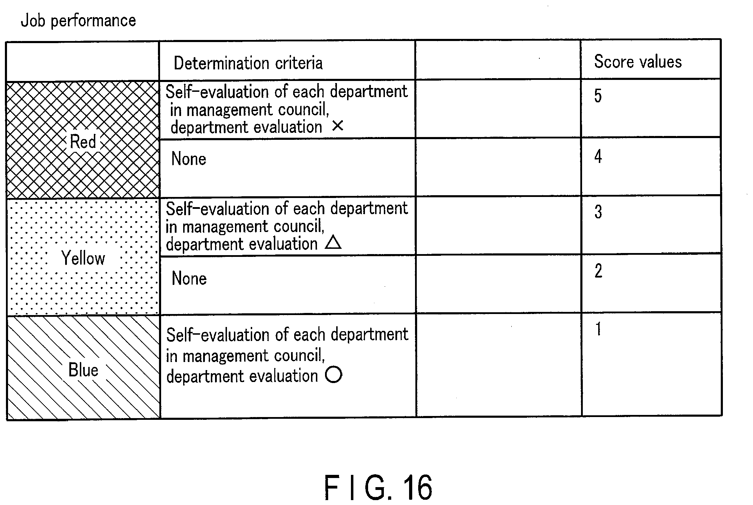

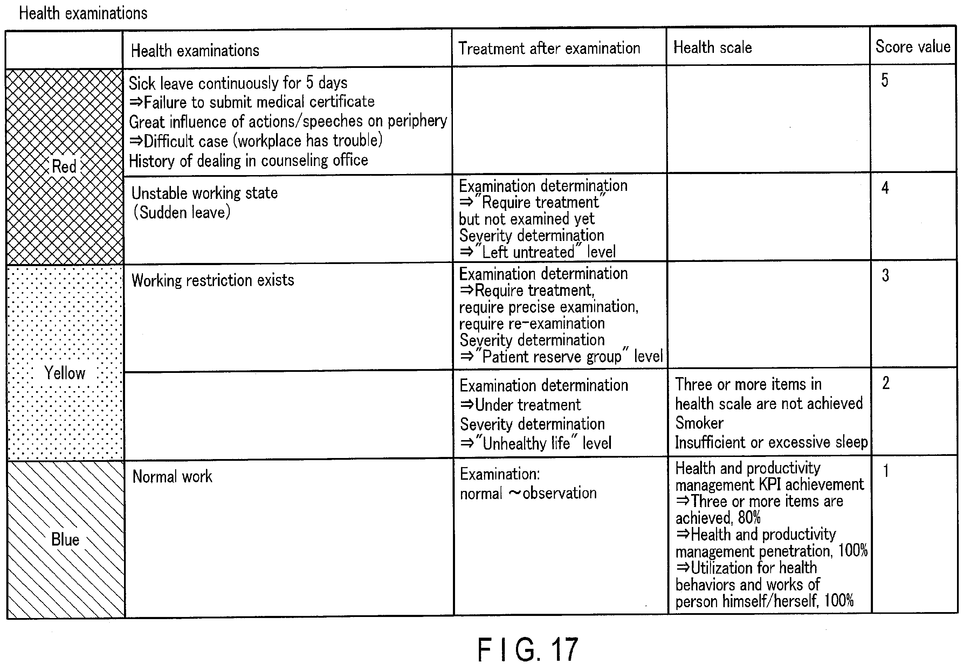

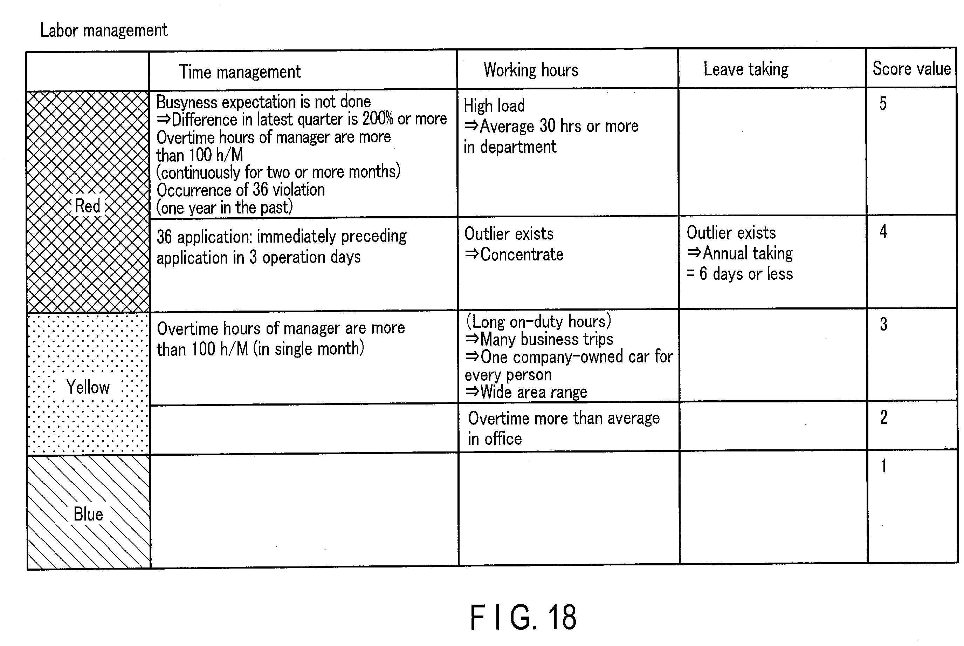

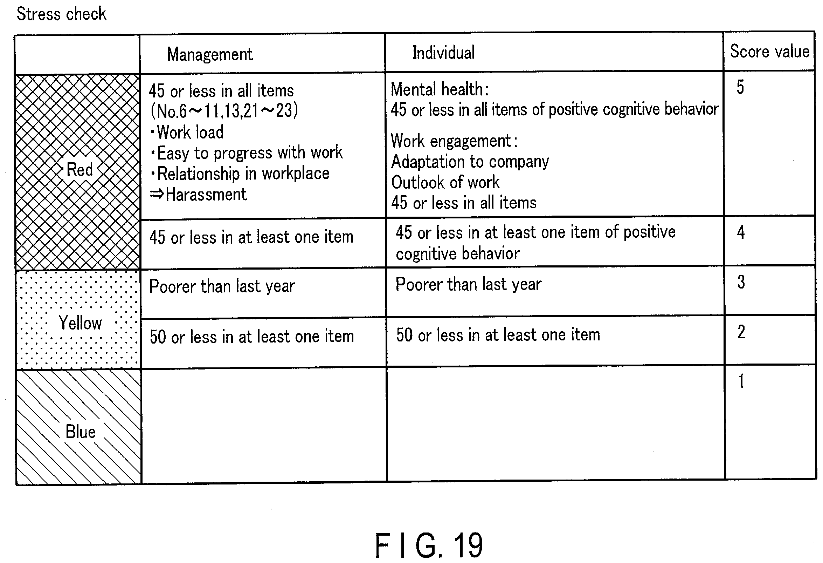

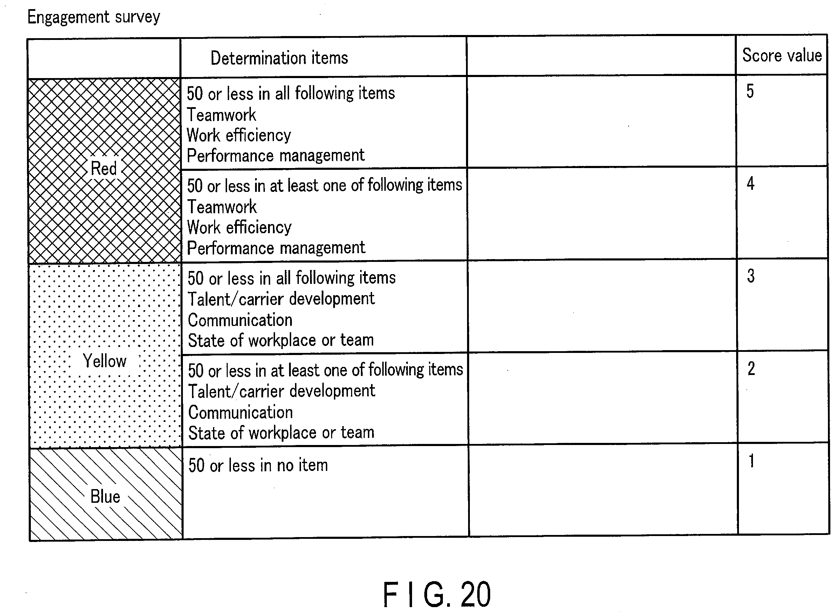

Examples of determination criteria are shown as tables in FIGS. 16, 17, 18, 19, and 20 on an index basis. The lower the score values shown in the tables are, the better the states to which the score values correspond are. In this embodiment, the score values are associated with colors, and a state becomes worse in the order of blue, yellow, and red. FIG. 16 shows determination criteria concerning the indices of job performance. Three determination items are shown, and score values are added in correspondence with these. FIG. 17 shows determination criteria concerning the indices of health examinations. Items of the contents of health examinations, measures based on determination after examinations, and health scales are shown, and score values are added in accordance with corresponding items. FIG. 18 shows determination criteria concerning the indices of labor management, and items of time management, working hours, and holiday acquisition are shown. Score values 2 to 5 are added if the determination criteria of the corresponding items are satisfied, and a score value 1 is added if none of the determination criteria is satisfied. FIG. 19 shows determination criteria concerning the indices of stress checks, and items of management and individual are shown. Score values 2 to 5 are added if the determination criteria of the corresponding items are satisfied, and a score value 1 is added if none of the determination criteria is satisfied. FIG. 20 shows determination criteria concerning the indices of engagement surveys. Five determination items are shown, and score values are added in accordance with corresponding items.

### (Step S1504)

In step S1504, the controller 201 calculates a score value for each index based on the result of comparison of the index contents of each index included in the index data in step S1503. In this embodiment, the score values are normally set in five levels from 1 to 5.

### [Operation Example: Step S604, Display Mode Setting Processing]

Processing of the display data generator 303 to set a display mode to display the evaluation values calculated in step S603 will be described next with reference to FIG. 21.

FIG. 21 is a flowchart showing an example of the processing procedure of step S604. Note that the processing procedure to be described below is merely an example, and each process may be changed as much as possible. Also, concerning the processing procedure to be described below, omission, replacement, and addition of steps can be done as needed in accordance with the embodiment.

### (Step S2101)

In step S2101, the controller 201 operates as the display data generator 303 (in the description of FIG. 21, the main operation component is the same and will be omitted below), and acquires information about the department and indices from the memory 202. As the information about the department, for example, characteristic data representing the characteristic of the department is acquired. The information about indices represents what kinds of indices are used. The characteristic data sets, for each department, association (for example, an association degree) between each index of the plurality of first indices and the second index (that is, job performance) based on the main business target, how to progress with works, the result of works, and the like in the department.

### (Step S2102)

In step S2102, the controller 201 decides a display area to display each index based on the information concerning the department and the characteristic data of the department, which are acquired in step S2101. The display data generator 303, for example, sets a large composition ratio for an index that largely contributes to improvement of job performance in all the indices, and sets a large display area as the composition ratio becomes large. In addition, since the index that is important for the improvement of job performance is different depending on the department, the display data generator 303 sets the composition ratios of indices for each department based on the characteristic data.

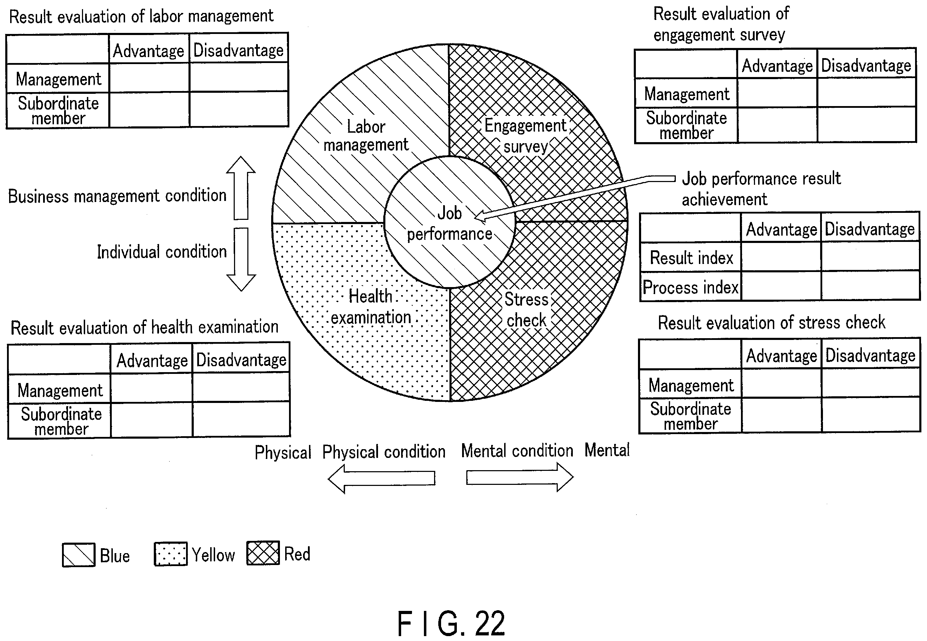

A pie chart shown in FIG. 22 is an example of display corresponding to the evaluation values of indices displayed based on the typical display mode of this embodiment. In the example shown in FIG. 22, the first indices are four indices including labor management, health examination, engagement survey, and stress check, and the second index is a job performance result. As for the first indices, a color representing the evaluation value of each index is added to a quarter disc in the pie chart. As for the second index, a color representing the evaluation value is added to a disc concentric to the pie chart indicating the first indices. The colors have the same meanings as the colors added to the score values determined based on the determination criteria shown in FIGS. 16, 17, 18, 19, and 20.

In addition, the vertical line of the pie chart corresponds to the axis for classifying the indices into physical conditions and mental conditions of human resource belonging to the organization. Labor management and health examination arranged on the left side of the axis correspond to the indices that reflect the physical conditions, and engagement survey and stress check arranged on the right side of the axis correspond to the indices that reflect the mental conditions. On the other hand, the horizontal line of the pie chart corresponds to the axis for classifying the indices into business management conditions and individual conditions in the organization. Labor management and engagement survey arranged on the upper side of the axis correspond to the business management conditions, and health examination and stress check arranged on the lower side of the axis correspond to the individual conditions. As described above, in this embodiment, the first indices are classified into four by the two axes. The number of indices classified by the first indices may be increased by adding an axis for classifying the indices by other conditions. That is, this embodiment corresponds to classifying the indices in two dimensions. However, indices that are classified in multi-dimensions including three or more dimensions may be used.

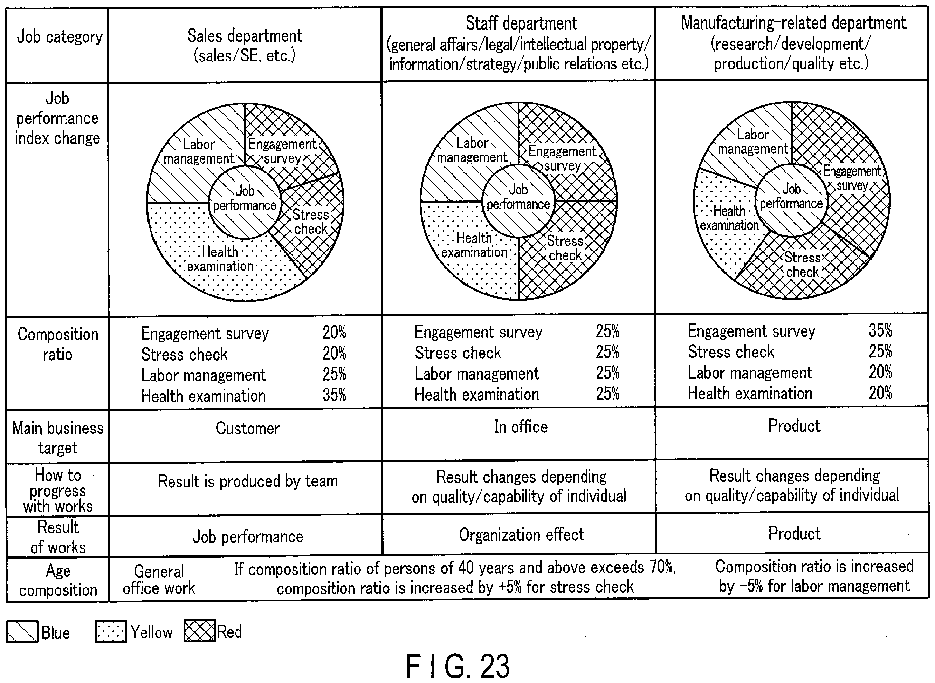

The composition ratios will be described next with reference to FIG. 23. FIG. 23 shows examples of three departments, and the main business target, how to progress with works, the result of works, and/or the age structure of each department are stored in the memory 202 as the characteristic data of the department. The display data generator 303 sets the composition ratios shown in FIG. 23 based on the characteristic data. For example, in the sales department, the physical conditions are important indices. The top priority is placed on the health examination, and labor management, engagement survey, and stress check follow. On the other hand, in the staff department, the four first indices have even composition ratios. In the manufacturing-related department, the most important index is engagement survey, and stress check, labor management, and health examination follow as the next higher composition ratios.

### (Step S2103)

In step S2103, the controller 201 reads out the type of a graph used to display the evaluation value for each index from the memory 202, and makes settings of display. In this embodiment, using a pie chart, index contents are displayed in regions each having a display area based on the composition ratio of each index, and an evaluation value obtained by evaluating each index is displayed in a corresponding region.

### (Step S2104)

In step S2104, the controller 201 reads out, from the memory 202, setting data concerning where to arrange the evaluation value of each index in the graph set in step S2103, and sets the place to arrange for each index. In this embodiment, which index is to be displayed where in the pie chart is set. In this embodiment, the first indices are arranged such that labor management is placed on the upper left side of the pie chart, and health examination, stress check, and engagement survey follow from there counterclockwise, as shown in FIG. 22 and subsequent drawings. In this embodiment, the second index is arranged as a disc at the center of the pie chart.

### (Step S2105)

In step S2105, the controller 201 reads out color data concerning colors corresponding to the evaluation values of the indices from the memory 202, and adds a color to each index in accordance with its evaluation value.

### (Step S2106)

In step S2106, the controller 201 decides the area to display the evaluation value of the second index in accordance with the evaluation value. In this embodiment, the display data generator 303 displays job performance that is the second index in a circle different from the first indices, and increases the display area in accordance with the job performance. In this embodiment, the circle indicating the second index is concentric to the center of the pie chart of the first indices and is shown with a radius smaller than the radius of the pie chart.

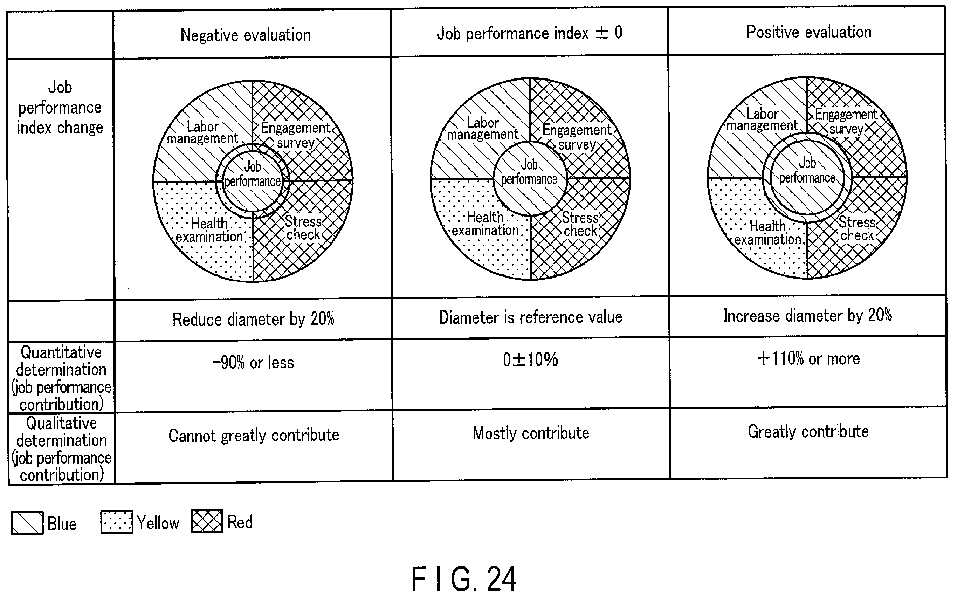

The display area of job performance is changed as in pie charts shown in FIG. 24. In the example shown in FIG. 24, using, as a reference, job performance that is evaluated neither negative nor positive, if the job performance is evaluated negative, the radius of the disc indicating the job performance is reduced, and if the job performance is evaluated positive, the radius of the disc indicating the job performance is increased. The reduction or increase ratio of the radius can arbitrarily be set, and an example is shown in FIG. 24.

### [Operation Example: Step S606, Support Data Generation Processing]

Processing of the support data generator 304 to generate support data based on the display data generated in step S605 will be described next with reference to FIG. 25.

FIG. 25 is a flowchart showing an example of the processing procedure of step S606. Note that the processing procedure to be described below is merely an example, and each process may be changed as much as possible. Also, concerning the processing procedure to be described below, omission, replacement, and addition of steps can be done as needed in accordance with the embodiment.

### (Step S2501)

In step S2501, the controller 201 operates as the support data generator 304 (in the description of FIG. 25, the main operation component is the same and will be omitted below), and acquires information about the department from the memory 202 and display data from the display data generator 303. As the information about the department, for example, the name of the department is acquired. The display data is shown in, for example, a pie chart as shown in FIGS. 22, 23, and 24.

### (Step S2502)

In step S2502, concerning the combination of the display data of the job performance as the second index and the labor management, health examination, engagement survey, and stress check as the first indices, the controller 201 refers to a table stored in the memory 202, in which the combination of display data and the problem categories are set, and selects a problem category to which the combination of display data belongs.

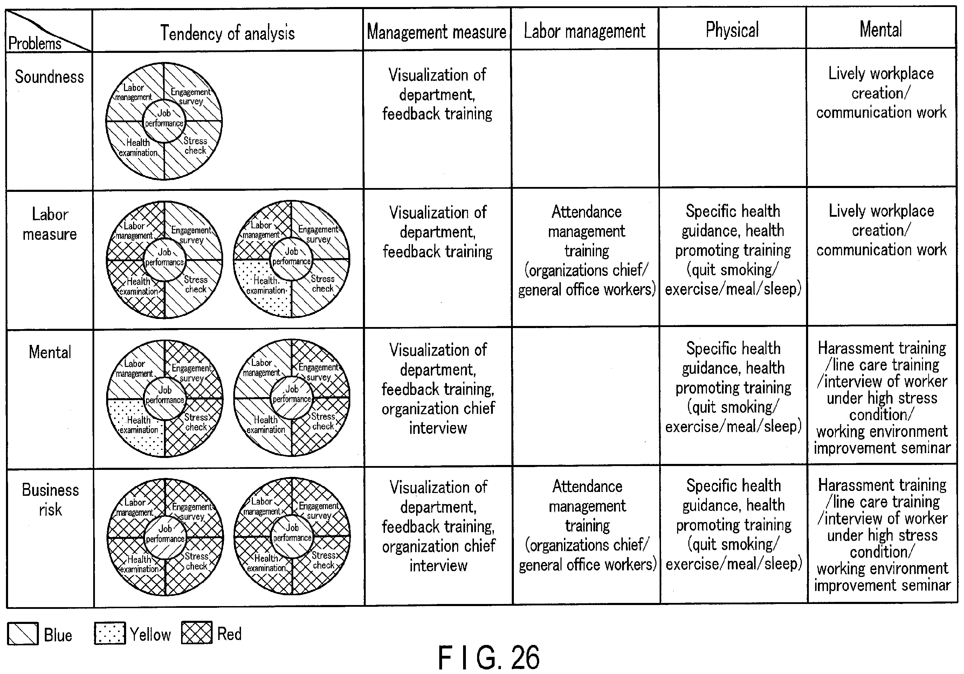

Four items vertically shown on the left side of FIG. 26 are examples of problem categories, and the items are classified into four categories here. That is, in the example shown in FIG. 26, Four categories including soundness, labor measure, mental, and business risk are shown. These categories are decided based on the distribution of the evaluation values of the first indices and the second index (or the distribution of the added colors).

### (Step S2503)

In step S2503, the controller 201 refers to the table of the combination of display data and the problem categories, which is stored in the memory 202, based on the problem category selected in step S2502, and sets a support measure based on the selected problem category.

FIG. 26 shows an example of the table. According to this table, four items shown on the leftmost side are the problem categories, and a support measure corresponding to each problem is presented for each of the four first indices shown in the uppermost portion.

### (Step S2504)

In step S2504, the controller 201 creates support data including the support measure set in step S2503.

By the table shown in FIG. 26, one of the four problem categories is decided based on the distribution of the pie chart decided by the evaluation values of the five indices (the tendency of the distribution shown in FIG. 26). Data including support measures corresponding to the four first indices corresponding to the problem is the support data.

Examples of support measures will be described below with reference to the accompanying drawings.

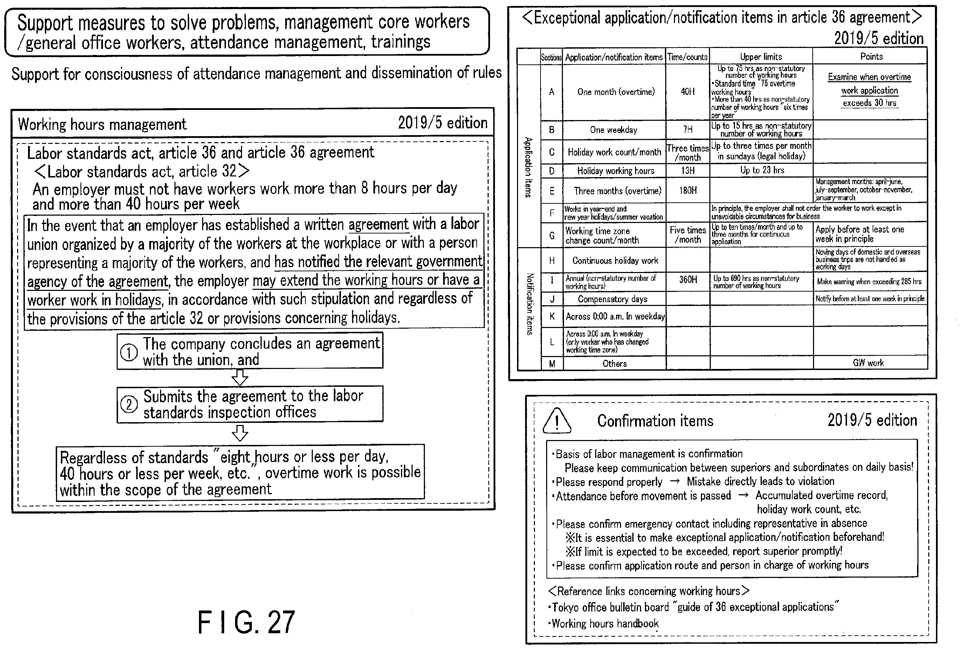

FIG. 27 shows an example of support measures concerning labor management. FIG. 27 shows support measures for consciousness of attendance management and dissemination of rules for managers. FIG. 27 particularly shows working hour management in detail.

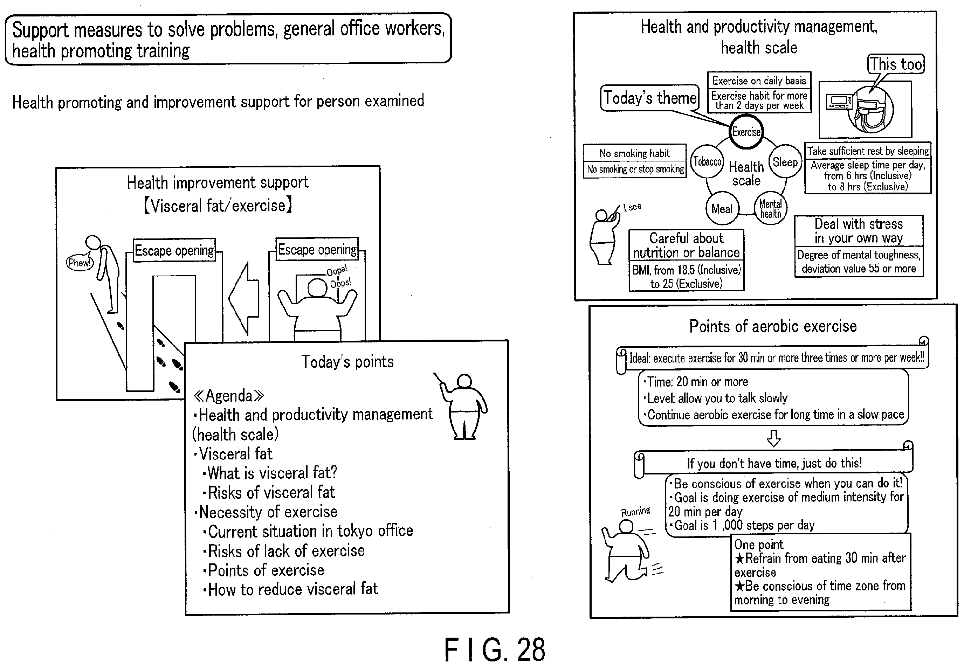

FIG. 28 shows an example of support measures concerning physical (similar to health examination). FIG. 28 shows specific health guidance, health promoting training, and the like.

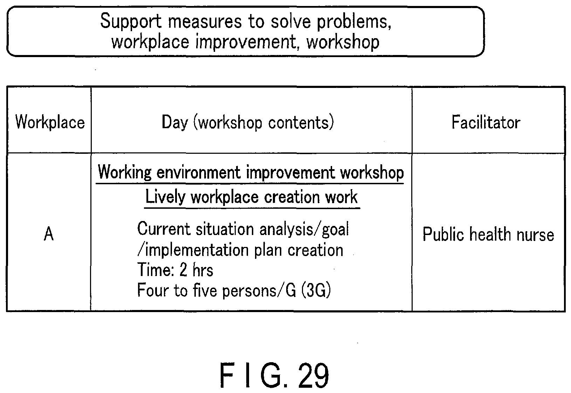

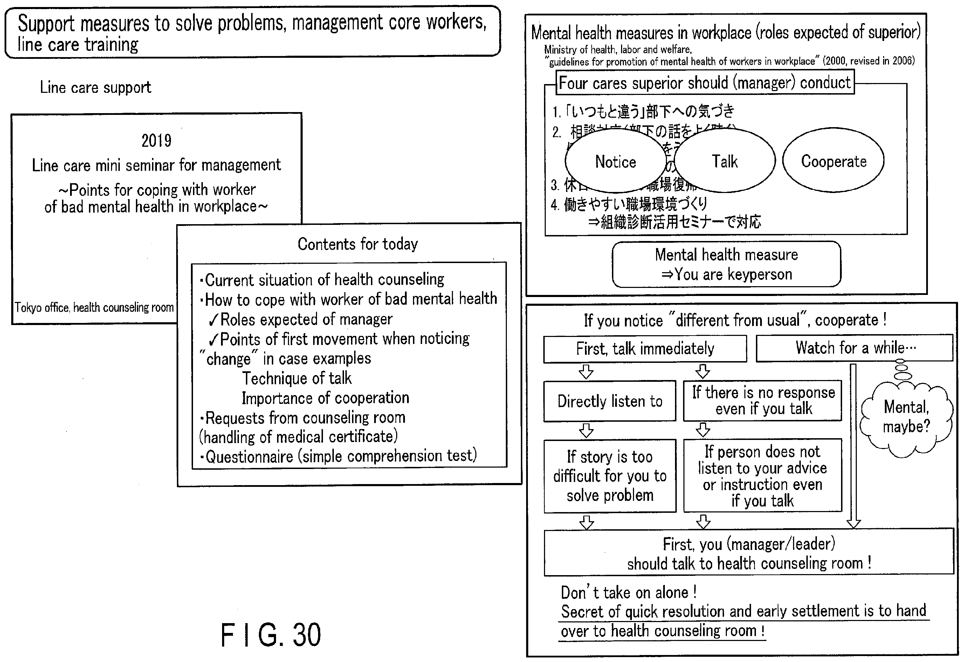

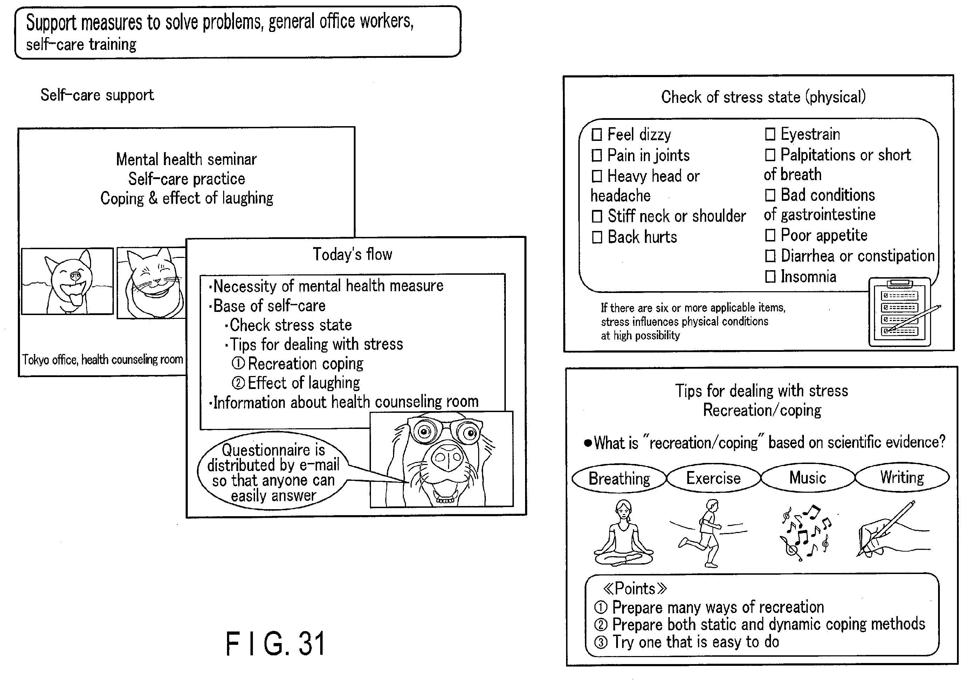

FIGS. 29, 30, and 31 show examples of support measures concerning mental (similar to engagement survey). FIG. 29 shows a state in which a workshop guide is presented. For example, a link is set to title "Working Environment Improving Workshop, Lively Workplace Creation Work", and a user can apply for participation to the workshop from the link destination. FIG. 30 shows an example of a line care support, and shows the contents of training concerning line care for managers. FIG. 31 shows an example of a self-care support, and shows the contents of training concerning mental health for each member of the organization.

### [Actions and Effects]

As described above, in step S602, the job performance support apparatus 101 according to this embodiment acquires index data including the contents of the plurality of first indices and the second index from the human resource belonging to the department. In steps S603, S1502, S1503, and S1504, determination criteria are loaded for each index, the index contents of each index and the determination criteria are compared, and an evaluation value is calculated for each index. As a result, it is possible to calculate evaluation values for all indices. In steps S604, S2102, S2103, S2104, S2105, and S2106, the composition ratios of the indices are set in accordance with the characteristic of the department, the type of a graph used to display the evaluation values of the indices is set, the positions to arrange the evaluation values and colors are set for the indices, and the area for the second index is set large as the evaluation value becomes large. It is therefore possible to generate display data in which the evaluation values of the indices are expressed by a graph representing the characteristic of each department. In steps S606, S2502, S2503, and S2504, a problem category is selected in correspondence with the combination of display data, and support data including support measures to support the human resource of the department is generated for each problem category. This makes it possible to appropriately extract the problem of the department and associate a corresponding support measure with the problem.

According to the above-described embodiment, it is possible to clearly understand, for each department, how the plurality of indices are related to the job performance of the second index. In addition, since it is possible to clearly show the problem of the department and present a support measure for the problem, it is possible to execute a detailed and appropriate measure for each personnel of the department. As a result, it is possible to improve the job performance of the department and improve the job performance of the organization. In addition, the physical conditions and the mental conditions of the human resource can be guided to good conditions, and the business management conditions and the individual conditions can also be guided to good conditions. Hence, the job performance of the organization can be improved, and the management conditions of the organization itself, the individual conditions of the members of the organization, and also the physical conditions and the mental conditions can be guided to good conditions. When display data in which a score value is associated with each index is time-serially listed, it is possible to easily and reliably analyze which index is improved or deteriorated in which manner. It is therefore possible to provide information for improving the job performance of the organization and support the members of the organization.

Also, by the indices based on the physical conditions and the mental conditions, and the business management conditions and the individual conditions, a criterion for the so-called degree of soundness or degree of health of the organization can be obtained, and information concerning how much the degree of soundness or the degree of health of the organization influences the job performance can be obtained from a plurality of aspects based on the evaluation values of the indices. Pieces of information held by an office or the like corresponding to the department are combined, and the result is replaced with a numerical value for each department, thereby making the performance of each department of the organization visible (visualize). Hence, data used to actively address improvement of job performance and improvement of productivity can be provided to the members of the organization. As a result, the departments and the organization can maximize the improvement activities, a workplace supporting force can be provided for each department, and a large effect (that is, job performance improvement) can be provided in the viewpoint of management.

### [Modifications]

The embodiment of the present invention has been described above in detail. However, the above description is merely an example of the present invention in all respects. Various changes and modifications can be made without departing from the scope of the present invention, as a matter of course. For example, the following changes are possible. In addition, when implementing the present invention, a detailed configuration according to the embodiment may appropriately be employed. Note that hereinafter, the same reference numerals as in the above embodiment denotes the same constituent elements, and a description of the same points as in the above embodiment will be omitted. The following modifications can appropriately be combined.

<1> The terminal apparatus 102 may store the program installed in the job performance support apparatus 101, acquire the index data of the first indices and the second index from the input device 406 directly or via the job performance support apparatus 101, and create the display data and the support data using the program.

Also, instead of presenting data by the terminal apparatus 102, the output device 207 of the job performance support apparatus 101 may present the display data, the support data, and the like, or setting may be done such that both the job performance support apparatus 101 and the terminal apparatus 102 can present the data.

<2> Each apparatus according to the present invention can be implemented by a computer and a program. The program can be recorded in a recording medium (or a storage medium) or provided via a network.

The above-described apparatuses and device portions thereof can be implemented by any of a hardware configuration and a combined configuration of a hardware resource and software. As the software of the combined configuration, a program is used, which is installed in advance from a network or a computer-readable recording medium (or storage medium) into the computer, and executed by the processor of the computer, thereby causing the computer to implement the operation (or function) of each apparatus.

<3> Note that the display data is not limited to the above-described embodiment, when implementing, the constituent elements can be modified and embodied without departing from the scope of the invention. Also, various inventions can be formed by appropriately combining a plurality of constituent elements disclosed in the embodiment. For example, several constituent elements may be removed from all the constituent elements shown in the embodiment. In addition, constituent elements across different embodiments may appropriately be combined.

In addition, "and/or" means one or more arbitrary items in items connected by and/or. As a detailed example, "x and/or y" means one of elements in a set {(x), (y), (x,y)} formed by three elements. As another detailed example, "x, y, and/or z" means one of elements in a set {(x), (y), (z), (x,y), (x,z), (y,z), (x,y,z)} formed by seven elements.

### (Supplementary Note 1)

There is provided a job performance support apparatus (101) comprising:
an acquisition unit (301) configured to acquire, for each department, index data including index contents corresponding to a plurality of first indices classified by at least two axes including an axis for classifying indices into a physical condition and a mental condition of a human resource belonging to an organization including at least one department, and an axis for classifying indices into a business management condition and an individual condition in the organization, and a second index concerning a job performance result of the department;
a calculation unit (302) configured to calculate, for each index, an evaluation value that evaluates the index contents from the index data;
a first generation unit (303) configured to designate a display mode of the evaluation value for each of the first indices and the second index, and generate, for each index, display data with which the evaluation value is associated; and
a second generation unit (304) configured to generate, for each department, support data including support measures corresponding to the first indices in accordance with a combination of the display data for the plurality of first indices and the second index.

### REFERENCE SIGNS LIST

- 101: job performance support apparatus
- 102: terminal apparatus
- 103: network
- 201: controller
- 202: memory
- 203: power supply unit
- 204: timing device
- 205: communication interface
- 206: input device
- 207: output device
- 208: external interface
- 301: index data acquisition unit
- 302: evaluation value calculation unit
- 303: display data generator
- 304: support data generator
- 401: controller
- 402: memory
- 403: power supply unit
- 404: timing device
- 405: communication interface
- 406: input device
- 407: output device
- 408: external interface
- 501: instruction data acquisition unit
- 502: data acquisition unit
- 503: data presentation unit

## Claims

1. A job performance support apparatus comprising:
an acquisition unit configured to acquire, for each department, index data including index contents corresponding to a plurality of first indices classified by at least two axes including an axis for classifying indices into a physical condition and a mental condition of a human resource belonging to an organization including at least one department, and an axis for classifying indices into a business management condition and an individual condition in the organization, and a second index concerning a job performance result of the department;
a calculation unit configured to calculate, for each index, an evaluation value that evaluates the index contents from the index data;
a first generation unit configured to designate a display mode of the evaluation value for each of the first indices and the second index, and generate, for each index, display data with which the evaluation value is associated; and
a second generation unit configured to generate, for each department, support data including support measures corresponding to the first indices in accordance with a combination of the display data for the plurality of first indices and the second index.

2. The apparatus according to claim 1, wherein the acquisition unit employs labor management and acquires labor management data as the first index reflecting the physical condition and the business management condition in the index data, employs a health examination and acquires health examination data as the first index reflecting the physical condition and the individual condition, employs an engagement survey and acquires engagement data as the first index reflecting the mental condition and the business management condition, and employs a stress check and acquires stress check data as the first index reflecting the mental condition and the individual condition.

3. The apparatus according to claim 1 or 2, wherein the evaluation value is set in accordance with a determination item corresponding to each index, and the calculation unit evaluates the index data based on the determination item and calculates the evaluation value.

4. The apparatus according to claim 3, wherein a determination criterion of the evaluation value is set for each determination item, and the calculation unit calculates the evaluation value by comparing the index data with the determination criterion.

5. The apparatus according to any one of claims 1 to 4, wherein the first generation unit designates the display mode such that a graph to which a color corresponding to the evaluation value is added for each index is arranged at a position set for each index.

6. The apparatus according to any one of claims 1 to 5, wherein the first generation unit sets, based on characteristic data representing a characteristic of each department, a composition ratio of each index of the first indices to all the first indices in correspondence with the department, and designates the display mode of the first index based on the composition ratio.

7. The apparatus according to any one of claims 1 to 5, wherein the first generation unit sets an area to display the display mode corresponding to the evaluation value of the second index in accordance with the evaluation value.

8. The apparatus according to any one of claims 1 to 7, wherein the second generation unit sets training data including training contents corresponding to each of the first indices as the support measure based on the combination of the display data.

9. The apparatus according to any one of claims 1 to 8, further comprising a presentation unit configured to present the display data and the support data according to the combination.

10. The apparatus according to claim 9, wherein the presentation unit further presents associated data associated with the display data of each index.

11. A job performance support method comprising:
acquiring, for each department, index data including index contents corresponding to a plurality of first indices classified by at least two axes including an axis for classifying indices into a physical condition and a mental condition of a human resource belonging to an organization including at least one department, and an axis for classifying indices into a business management condition and an individual condition in the organization, and a second index concerning a job performance result of the department;
calculating, for each index, an evaluation value that evaluates the index contents from the index data;
designating a display mode of the evaluation value for each of the first indices and the second index, and generating, for each index, display data with which the evaluation value is associated; and
generating, for each department, support data including support measures corresponding to the first indices in accordance with a combination of the display data for the plurality of first indices and the second index.

12. A program configured to cause a computer to function as each unit provided in a job performance support apparatus defined in any one of claims 1 to 10.
